# EUROPEAN PATENT APPLICATION

(11) **EP 1 661 511 A1**
(43) Date of publication of application: **31.05.2006**
(21) Application number: 04772884.5
(22) Date of filing: 01.09.2004
(51) Int. Cl.: A61B 5/0245, A61B 5/08, A61B 5/11

(54) **BIOLOGICAL SENSOR AND SUPPORT SYSTEM USING THE SAME**

(30) Priority: 02.09.2003 JP 2003309797; 24.09.2003 JP 2003331639; 25.09.2003 JP 2003333857
(71) Applicant: MATSUSHITA ELECTRIC INDUSTRIAL CO., LTD., Kadoma-shi, Osaka 571-8501 (JP)
(72) Inventor: OGINO, Hiroyuki, Nara-shi, Nara 630-8024 (JP); UEDA, Shigeki, Nara 630-1055 (JP); UMEKAGE, Yasuhiro, Shida 520-3035 (JP)
(74) Representative: Grünecker, Kinkeldey, Stockmair & Schwanhäusser Anwaltssozietät
(86) International application number: PCT/JP2004/013012
(87) International publication number: WO 2005/023105

(57) **Abstract**

It is an object of the invention to provide a biological sensor capable of detecting a biological information with a high sensitivity. It is an object of the invention to provide a support system for carrying out a support in such a manner that a work can be efficiently performed based on the biological information obtained by the biological sensor.

There are provided acceleration detecting means (300) constituted to detect an acceleration including a vibration of a body surface of a person to be measured, processing means for extracting at least two of an information about a heartbeat, an information about a body motion and an information about a breath from an information output from the acceleration detecting means (300), and deciding means (500) for deciding a condition of the measured person to be a living body from an output of the processing means.

## Description

### <Technical Field>

The present invention relates to a biological sensor and a support system using the same and more particularly to a system for detecting the condition of a living body from an information about a heartbeat, an information about a body motion and an information about a breath and supporting a living body in various scenes of a daily life such as a business support, a nursing support and a child care support.

### <Background Art>

As a serious aspect for supporting a high growing society, people make such an environment that they can sleep comfortably, can spend time comfortably and can work efficiently depending on the condition of a living body. Moreover, this is also applied to people living in special environments such as babies, the aged and sick people in addition to healthy adults. If it is possible to objectively detect the various conditions and to support the people to live more comfortably depending on the conditions, it is possible to implement more complete child care, care and nursing supports, thereby approaching a happier aging society. Herein, the condition of the living body (a biological condition) implies the state of a mind and body which has no will, for example, a degree of concentration, a degree of awakening, a degree of fatigue, a degree of tension and a degree of discomfort.

Usually, a person can make other people understood through information transmitting means such as a language and can thus change an environment surrounding him (her). In many cases, however, it is possible to spend time more comfortably by objectively grasping a biological information and changing an environment to create a better environment irrespective of an intention or in a scene having no intension.

For example, in an office, people feel tired and it is hard to afford to take countermeasures by themselves in many cases. Consequently, the fatigue is often accumulated. If a degree of fatigue can be detected objectively by means of a sensor, however, it is possible to prevent the accumulation of the fatigue from causing a reduction in an efficiency and a disease by taking measures, for example, supplying oxygen at an early stage corresponding to the degree of fatigue, feeding a healing scent through an aromatherapy or giving music to carry out healing.

In the case in which a person feels sleepy in the office, moreover, it is possible to prevent a nap and to increase the efficiency of a work by objectively detecting the feeling of sleep and supplying the oxygen to him (her) from a machine or feeding the oxygen.

In such a situation, a sensor capable of objectively grasping the condition of a living body has been desired to be introduced.

Conventionally, there has also been proposed a method of detecting the vibration of a living body by vibration detecting means, thereby measuring a heartbeat.

For example, a method of measuring a heartbeat by arranging a sensor under an elastic member portion such as the urethane of a car seat has been described in the prior application related to the applicant (see Patent Document 1, for example).
(Patent Document 1) JP-A-8-282358 Publication

In this method, vibration detecting means is fixed onto a seat spring constituting a seat provided apart from the contact surface of the seat and a human body at a constant distance or more through a front cloth or an urethane foam. Herein, the vibration detecting means is constituted to be provided apart from the human body taking a seat at such a distance that the presence of the vibration detecting means is not felt, and is shortened if the hardness of a member covering the vibration detecting means is increased. Accordingly, the vibration detecting means becomes longer if the urethane foam is softer.

Thus, the vibration detecting means is fixed to a portion provided apart from the surface of the seat formed of the urethane at a constant distance or more and the output of the vibration detecting means is processed to decide the presence of the human body on the seat through the output. The vibration detecting means is provided apart from the contact surface with the human body. Even if the vibration detecting means is constituted by a rigid member, therefore, it is possible to lessen an influence on a feeling of seating.

In such a sensor, however, a sufficient output cannot be obtained, it is possible to decide at least the presence of a human body, and the biological information cannot be detected objectively. Moreover, there is no thought to detect a plurality of biological informations such as a breath, a heartbeat and a body motion from the output of the vibration detecting means, and it is impossible to consider such a development as to give a living support for a living body to live comfortably based on the biological informations.

The invention has been made in consideration of the actual circumstances and has an object to provide a biological sensor capable of detecting a biological information with a high sensitivity.

Moreover, it is an object of the invention to provide a work support system for carrying out a support in order to perform a work efficiently based on the biological information obtained from the biological sensor.

Furthermore, it is an object of the invention to provide a living support system for supporting a live in order to implement a more comfortable life based on the biological information obtained from the biological sensor.

In the operation of a vehicle, moreover, the biological condition of a driver influences a driving stability. The biological condition implies the state of a mind and body having no intention, for example, a degree of concentration, a degree of awakening, a degree of fatigue, a degree of tension, a degree of discomfort and a physical condition. For this reason, a method of detecting the biological condition of the driver such as a nap condition has been studied in order to support the driver to safely drive the vehicle. For a technique of this type, there has been known a biological information measuring device for detecting a heart rate in order to know the physical condition of the driver (see Patent Document 2, for example).

The conventional biological information measuring device comprises an operation fixing portion (a seat belt) for fixing the motion of a person to be inspected, a vibration measuring portion for measuring a heartbeat, and a vibration measuring position adjusting portion for aligning the vibration measuring portion with the position of the chest of the inspected person. By this structure, when the inspected person simply attaches the seat belt to align the portions with each other, an electrocardiac R wave can be measured.

In the conventional biological information measuring device, however, there is a possibility that the very small vibration of a heartbeat cannot be detected accurately by the influence of the vibration of a vehicle.
(Patent Document 2) JP-A-2001-8922 Publication

The invention has been made in consideration of the circumstances and has an object to provide a biological condition deciding device capable of detecting a biological information with a high sensitivity and high precision and objectively deciding the biological condition of a person to be measured. Moreover, it is an object of the invention to provide a system for supporting the measured person to spend time comfortably by using the biological condition deciding device.

As the conventional biological information detecting device, moreover, there has been proposed a device for detecting a micro vibration signal based on the heartbeat of a human body to be a living body, thereby detecting an information about the heartbeat of the living body. This serves to cause a human body to carry out a very small vibration at a resonant frequency (approximately 4 to 7 Hz) by the heartbeat activity of a heart and to detect the very small vibration which is synchronous with a heartbeat through a vibration sensor, thereby calculating and displaying the number of the heartbeats, and has an advantage that an electrode is not attached to a living body but the detection can be carried out without a restraint. Since a vibration signal which is synchronous with the heartbeat is minute, however, there is a problem in that the vibration signal which is synchronous with the heartbeat is influenced by a running vibration and the heartbeat cannot be detected if a vibration noise is made on an outside as in the case in which a car is running, for example. In order to solve the problem, there has been proposed a device including another vibration sensor and serving to output a heartbeat signal based on a difference between the output signals of the two vibration sensors (see Patent Document 3, for example).

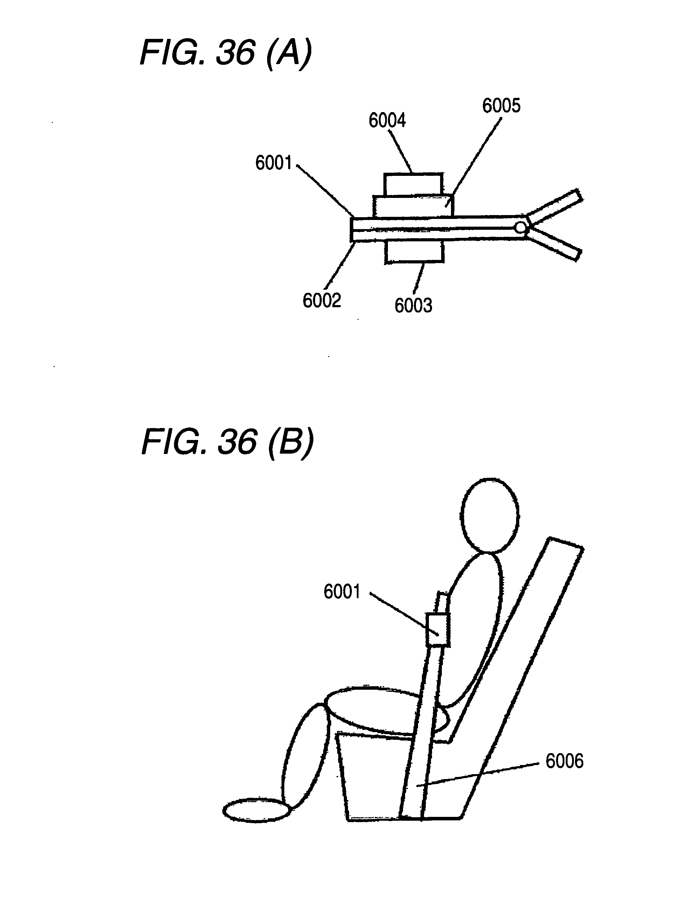

Figs. 36(a) and 36(b) show a conventional biological information detecting device described in the publication. As shown in Fig. 36(a), a conventional biological information detecting device 6001 is constituted by a clip 6002, a first acceleration sensor 6003, a second acceleration sensor 6004, and a medium 6005 having a hallow portion therein and formed of a resin. The medium 6005 has such a property as to attenuate the vibration frequency component of a living body through a heartbeat. The biological information detecting device 6001 is attached to a seat belt 6006 of a car by means of the clip 6002 as shown in Fig. 36(b). In this case, the first acceleration sensor 6003 is positioned on a body side.

By the structure, while the vibration and the running vibration generated by the heartbeat are detected by the first acceleration sensor 6003, the vibration generated by the heartbeat is attenuated through the medium 6005 and only the running vibration is detected by the second acceleration sensor 6004. Therefore, the difference between the output signals of the first acceleration sensor 6003 and the second acceleration sensor 6004 is taken to detect only the vibration signal generated by the heartbeat.
(Patent Document 3) JP-A-4-5950 Publication

With the conventional structure, however, a human body is vibrated slightly by a heartbeat, and at the same time, the human body is also vibrated by a running vibration and the human body is vibrated at a resonant frequency (approximately 4 to 7 Hz) of the human body. Therefore, the minute vibration of the human body which is generated by the heartbeat is buried in the great vibration of the human body which is generated by the running vibration.

When a sensor output is actually observed, the running vibration and the vibration of the human body which is generated by the running vibration are detected by the first acceleration sensor 6003. The medium 6005 attenuates the resonant frequency (approximately 4 to 7 Hz) of the human body. Consequently, the great vibration of the human body which is generated by the running vibration is attenuated by the medium 6005 so that the running vibration is detected by the second acceleration sensor 6004.
Therefore, there is a problem in that the great vibration signal of the human body is simply obtained by the running vibration even if the difference between the output signals of the first acceleration sensor 6003 and the second acceleration sensor 6004 is taken, and therefore, the vibration signal generated by the heartbeat is buried in the great vibration signal of the human body and is hard to detect.

Moreover, the transfer of the vibration through the medium 6005 is delayed in a process for propagating the running vibration to the first acceleration sensor 6003 and the second acceleration sensor 6004 through the seat belt 6006. Consequently, a phase difference is made on the output signals of the first acceleration sensor 6003 and the second acceleration sensor 6004. With the structure, however, the phase difference is not considered in the case in which the difference between the output signals is to be taken. For this reason, there is a problem in that a running vibration component remains in a signal obtained by the difference.

In order to solve the conventional problems, it is an object of the invention to provide a biological information detecting device capable of detecting an information about the heartbeat of a living body even if a vibration noise is made on an outside.

### <Disclosure of the Invention>

In order to achieve the object, a biological sensor according to the invention comprises acceleration detecting means constituted to detect an acceleration including a vibration of a body surface of a person to be measured, and deciding means for extracting at least two of an information about a heartbeat, an information about a body motion and an information about a breath from an information output from the acceleration detecting means and deciding a condition of the measured person to be a living body.

By this structure, at least two of the information about a heartbeat, the information about a body motion and the information about a breath are extracted, thereby deciding the condition of the living body. Therefore, the condition of the living body can be objectively detected with high precision and this information can be used for various biological support systems.

Moreover, the invention provides the biological sensor further comprising storage means for storing the information output from the acceleration detecting means, the deciding means setting the information output from the acceleration detecting means which is stored in the storage means to be a reference information and deciding the biological condition in consideration of the reference information.

By this structure, if the output information obtained by the acceleration detecting means is prestored in the storage means and the condition of the living body is decided in consideration of the data thus stored, the characteristics of the acceleration detecting means itself can also be cancelled, the condition of the living body can be decided more efficiently, and it is possible to prevent an erroneous decision in which an output based on the characteristics of the acceleration detecting means itself is decided to be caused by the living body.

Furthermore, the invention provides the biological sensor, wherein the storage means serves to store the information output from the acceleration detecting means in a usual condition of the measured person, and the deciding means serves to decide the biological condition of the measured person by referring to the output information of the storage means.

Consequently, it is possible to make a decision by referring to an information in a usual condition of the measured person himself (herself). Therefore, it is possible to efficiently make a more accurate decision.

In addition, the invention provides the biological sensor, wherein the acceleration detecting means includes an elastic member having a low repulsion and a piezoelectric cable sensor laid on the elastic member.

Consequently, it is possible to carry out the detection with high precision.

Moreover, the invention provides the biological sensor, wherein the acceleration detecting means is a piezoelectric cable sensor attached to a keyboard of a personal computer and laid on a palm rest provided in such a manner that at least a part of a palm of the measured person comes in contact therewith.

By this structure, the piezoelectric cable sensor is provided in the so-called palm rest which is disposed between the keyboard and a user and serves to cause the wrist of the user to take a rest in a personal computer input. Consequently, an input person (a person to be measured) does not have a feeling of incompatibility but a detection can be carried out more reliably with high precision in the same manner as in a usual input. Moreover, independent sensors are provided in left and right parts. By detecting informations about both left and right hands respectively and carrying out a processing based on the two informations, therefore, it is possible to detect a biological information with higher precision. By incorporating the biological sensor into the decision of the operation of a screen saver, furthermore, it is possible to detect, by means of the biological sensor, whether the input person thinks, leaves a seat or takes a nap when a predetermined time passes without the input person carrying out a keyboard input and the screen saver is operated, for example. In the case in which the thinking is continuously carried out even if the time passes without the keyboard input, therefore, it is possible to prevent the occurrence of a bad influence that the keyboard input dare to be carried out to disturb the thinking or the screen saver is operated to disturb the thinking if the operation of the screen saver is controlled to leave the operation of the screen saver. Moreover, it is possible to support to progress the thought of the input person more smoothly and efficiently by supplying a fresh scent interlockingly with an oxygen supply system or an aromatherapy system in addition to the control of the operation of the screen saver at this time. Also in the case in which the input person is present or takes a nap, furthermore, the operation of the oxygen supply system or the aromatherapy system may be executed.

Furthermore, the invention provides the biological sensor, wherein the acceleration detecting means is a piezoelectric cable sensor laid on a seat portion.

By this structure, it is possible to reliably detect a biological information such as an information about a heartbeat by using a seat portion which can be bent freely and can be provided everywhere without the limitation of an installation place, and has a comfort to sit on without a feeling of incompatibility.

In addition, the invention provides the biological sensor, wherein the acceleration detecting means is a piezoelectric cable sensor laid on a pendant to be hung around a neck of the measured person.

By this structure, it is possible to reliably detect a biological information such as an information about a heartbeat without a feeling of incompatibility for the measured person. Moreover, the acceleration detecting means may be fixed to the head portion of the pendant.

Furthermore, the invention provides the biological sensor, wherein the acceleration detecting means is a piezoelectric cable sensor laid on a ring-shaped member formed to be attachable to the measured person.

By this structure, it is possible to reliably detect a biological information such as an information about a heartbeat. The ring-shaped member may be incorporated into a bracelet to be attached to the arm of the measured person or a belt to be attached to the waist part of the measured person. In this case, the action of the measured person is not restricted if a signal fetched from the ring-shaped member can be transmitted by wireless to a processing device such as a personal computer.

In addition, the invention provides the biological sensor, wherein the acceleration detecting means is a piezoelectric cable sensor laid on a bed.

By this structure, the piezoelectric cable sensor is used. Therefore, it is possible to reliably detect a heartbeat vibration without a feeling of incompatibility for the measured person.

If the bed pad is constituted by an elastic member having a low repulsion such as low repulsive urethane, an ordinary cushion layer which is laid over the lower surface of the low repulsive urethane layer and is formed of urethane, and a piezoelectric cable sensor laid over the lower surface of the cushion layer, moreover, a heartbeat vibration can be detected more reliably, and furthermore, a comfort to sleep can also be increased without a feeling of incompatibility. By constituting the piezoelectric cable sensor with a flexible cable sensor, furthermore, it is possible to detect the heartbeat vibration more reliably. By mounting such a bed pad on a rigid plate having concavo-convex portions on a surface, moreover, it is possible to detect the heartbeat vibration more reliably.

By mounting the piezoelectric cable sensor on a movable plate attached onto a bed frame tiltably, moreover, it is possible to detect the heartbeat vibration reliably and to obtain a bed for care which has a comfort to sleep.

In addition, the invention provides the biological sensor, wherein the acceleration detecting means is a piezoelectric cable sensor laid on a toilet seat in a bathroom.

By this structure, it is possible to objectively detect the condition of the measured person to be a living body from a biological information such as a heartbeat, a body motion and a breath of the measured person. In the case in which the measured person is aged or sick, therefore, a person practicing medicine or family can reliably decide a situation and can make a reliable and objective decision without depending on a representation such as a language or a facial expression. Consequently, it is possible to implement a proper service more reliably. For example, the aged or sick people often express a different intension from a fact due to a modesty or a prejudice. For instance, they say "no desire to urinate" for the question of a nursing person (a person for giving a service) due to a feeling such as a modesty or a shame even if they have the desire to urinate in many cases. However, the nursing person can accurately grasp the condition of a living body based on the objective biological information in addition to a response and can implement an optimum service.

Moreover, the invention provides an autonomic nerve training system using the biological sensor, wherein the deciding means serves to detect an autonomic nerve condition from at least two of the heartbeat, the body motion and the breath, the autonomic nerve training system further comprising display means for displaying the autonomic nerve condition as an information output from the deciding means in such a manner that the trainee carries out an autonomic nerve training while seeing a display of the display means.

By this structure, it is possible to detect the autonomic nerve condition of a living body based on the objective biological information. Therefore, it is possible to promote the consciousness of the trainee and to implement a more efficient training. For example, if the trainee to carry out a vigilance conquest training can recognize that he (she) is brought in a close condition to a sleep as a biological information, a sense of security can be given to him (her) and he (she) is guided to the sleep more efficiently. In this case, the biological sensor may be provided in or attached to a pillow or a bed, an information about a twilight or a starry sky may be displayed on a ceiling or the color of a light may be changed by a light representation as display means, or an information may be displayed based on an information about music by using acoustic means, and it is desirable to use a method capable of causing the trainee to carry out a recognition comfortably.

Furthermore, the invention provides an aromatherapy system using the biological sensor, wherein the deciding means serves to detect a degree of tension as an output information, and a perfume is selected by referring to an aromatherapy information depending on the degree of tension, thereby relieving a biological condition.

By this structure, the degree of tension can be grasped objectively and the aromatherapy system can be operated automatically. Therefore, it is possible to carry out a support to give a more comfortable life.

In addition, the invention provides a sound system using the biological sensor, wherein the deciding means serves to detect a degree of tension, the sound system comprising music supply means for selecting and supplying proper music depending on the degree of tension.

By this structure, the degree of tension can be grasped objectively and the sound system can be operated automatically. Therefore, it is possible to carry out a support to give a more comfortable life.

Moreover, the invention provides a nap preventing system using the biological sensor, wherein the deciding means serves to detect a degree of awakening, the nap preventing system comprising support means for supporting awakening, and driving control means for controlling a driving operation of the support means depending on the degree of awakening.

By this structure, an operator (a measured person) can be awakened earlier in order to efficiently implement a job in an office. Consequently, it is possible to obtain a comfortable working environment. Furthermore, a manager can easily carry out various proper supports more effectively, for example, he (she) supplies music for a time period in which most operators want to sleep more greatly, carries out an aromatherapy or supplies oxygen.

In addition, the invention provides a nursing support system using the biological sensor, wherein the deciding means serves to detect a desire to urinate, the nursing support system comprising display means for displaying the output information for a nursing person.

As described above, by this structure, it is possible to implement a more precise care to produce high results by objectively grasping the desire to urinate as an information about the condition of a living body.

Moreover, the invention provides an input support system using the biological sensor, wherein the deciding means serves to detect a degree of awakening of a brain, and an activation of a screen saver is controlled based on the degree of awakening.

As described above, by this structure, it is possible to implement a more precise input support to produce high results by objectively grasping the degree of awakening as an information about the condition of a living body.

As described above, the biological sensor according to the invention is constituted to objectively grasp the condition of the living body based on the biological information such as a heartbeat, a body motion and a breath. Therefore, it is possible to implement a reliable and proper support.

Furthermore, the invention provides a biological condition deciding device for deciding a biological condition of a person to be measured based on an information output from a piezoelectric sensor having a flexibility, comprising a first piezoelectric sensor provided on a seat belt, a second piezoelectric sensor provided in a position placed apart from the measured person, and deciding means for deciding the biological condition of the measured person based on an information output from each of the first and second piezoelectric sensors, the first piezoelectric sensor serving to detect at least two of an information about a heartbeat, an information about a body motion and an information about a breath of the measured person and the second piezoelectric sensor serving to detect an information about a vibration of a vehicle.

By this structure, the deciding means decides the biological condition of the measured person based on the information output from each of the first and second piezoelectric sensors. Therefore, it is possible to detect the biological information with a high sensitivity and high precision and to objectively decide the biological condition of the measured person.

In addition, the invention provides the biological condition deciding device, wherein the first piezoelectric sensor is provided in a position on the seat belt which comes in contact with the measured person. By this structure, it is possible to accurately detect the biological information of the measured person by means of the first piezoelectric sensor.

Moreover, the invention provides the biological condition deciding device, wherein the first piezoelectric sensor is formed integrally with the seat belt. By this structure, the seat belt provided with the piezoelectric sensor can be attached without a feeling of incompatibility, which is practical.

Furthermore, the invention provides the biological condition deciding device, wherein the deciding means has a processing portion for processing the information output from each of the first and second piezoelectric sensors, the processing portion serving to carry out a frequency analysis over the output information. By this structure, also in the case in which a large number of noises are made on an outside as in running, for example, it is possible to detect an information in a predetermined frequency band by the frequency analysis. Moreover, it is possible to process an output information without taking a phase difference into consideration.

In addition, the invention provides the biological condition deciding device, wherein the processing portion has first calculating means for calculating respective power spectra of output signals of the first and second piezoelectric sensors, second calculating means for calculating, for each frequency, a difference between both of the power spectra calculated in the first calculating means, and third calculating means for obtaining, as an information about a heartbeat, a frequency at which the difference between the power spectra is maximized in a fundamental frequency region of the heartbeat which is preset based on a result of the calculation of the second calculating means.

Moreover, the invention provides the biological condition deciding device, further comprising storage means for storing the information output from each of the first and second piezoelectric sensors, the deciding means serving to decide the biological condition of the measured person by referring to an information stored in the storage means. By referring to a biological information about the measured person which is stored in the storage means, it is possible to accurately detect a change in the biological condition, thereby deciding the biological condition of the measured person precisely.

Furthermore, the invention provides the biological condition deciding device,
wherein the storage means serves to store the information output from the sensor in a usual condition of the measured person. By referring to a biological information in the usual condition of the measured person which is stored in the storage means, it is possible to accurately detect a change in a biological condition as compared with the usual condition, thereby deciding the biological condition of the measured person more precisely.

In addition, the invention provides the biological condition deciding device, wherein the first piezoelectric sensor is provided in a meandering form. By this structure, precision in the detection of the biological information can be enhanced.

Moreover, the invention provides a sound system using the biological condition deciding device according to any of claims 17 to 24, wherein the deciding means serves to decide a degree of tension of the measured person, the sound system comprising music supply means for selecting and supplying proper music depending on the degree of tension. With this structure, by giving proper music before the measured person himself (herself) is conscious of a tension condition, it is possible to relieve the tension of the measured person.

Furthermore, the invention provides a fatigue relaxing support system using the biological condition deciding device according to any of claims 17 to 24, wherein the deciding means serves to decide a degree of fatigue of the measured person, the fatigue relaxing support system comprising support means for supporting a relaxation of fatigue, and driving control means for controlling a driving operation of the support means depending on the degree of fatigue. By this structure, the support means is driven before the measured person himself (herself) is conscious of a fatigue condition. Therefore, it is possible to support the relaxation of the fatigue of the measured person.

In addition, the invention provides a nap preventing system using the biological condition deciding device according to any of claims 17 to 24, wherein the deciding means serves to decide a degree of awakening of the measured person, the nap preventing system comprising support means for supporting awakening, and driving control means for controlling a driving operation of the support means depending on the degree of awakening. By this structure, the support means is driven before the measured person himself (herself) is conscious of a reduction in the degree of awakening. Therefore, it is possible to prevent the measured person from taking a nap.

According to the invention, it is possible to provide a biological condition deciding device capable of detecting a biological information with a high sensitivity and high precision and objectively deciding the biological condition of the measured person. Moreover, it is possible to provide a system for supporting that the measured person spends time comfortably by using the biological condition deciding device.

Moreover, the invention provides a biological information detecting device, wherein power spectra of respective output signals of first vibration detecting means for detecting a vibration of a living body and second vibration detecting means provided separately through vibration attenuating means are calculated by first calculating means, a difference between both of the power spectra thus calculated is calculated for each frequency by second calculating means, and third calculating means obtains, as an information about a heartbeat, a frequency at which the difference between the power spectra is maximized in a fundamental frequency region of a heartbeat which is preset based on a result of the calculation of the second calculating means.

Consequently, in the case in which a running vibration is applied to a human body to be a living body, for example, the human body is slightly vibrated by a heartbeat, and at the same time, the human body is also vibrated by the running vibration and is vibrated at a resonant frequency (approximately 4 to 7 Hz) of the human body in the same manner as described above. Therefore, the minute vibration of the human body which is generated by the heartbeat is buried in the great vibration of the human body which is generated by the running vibration. However, the vibration signal of the human body at this time includes a vibration signal in the fundamental frequency region of a heartbeat usually appearing at approximately 1 to 2 Hz, and the vibration signal component is extracted by calculating a difference in a power spectrum between the respective output signals of the first vibration detecting means and the second vibration detecting means. More specifically, while the vibration signal in the fundamental frequency region of the heartbeat described above is detected by the first vibration detecting means, it is attenuated by the vibration attenuating means and is propagated to the second vibration detecting means. Therefore, it is possible to calculate the difference in the power spectrum between the respective output signals of the first vibration detecting means and the second vibration detecting means and to obtain, as an information about a heartbeat, a frequency at which the difference in the power spectrum is maximized in the fundamental frequency region of the heartbeat which is preset.

The information about a heartbeat is obtained by taking note of the fundamental frequency region of the heartbeat usually appearing at approximately 1 to 2 Hz in place of the frequency band (approximately 4 to 7 Hz) of the resonant frequency of a living body as in the conventional art. Differently from the conventional art, therefore, it is possible to prevent a heartbeat signal from being buried in the great vibration signal of the human body which is generated by the running vibration, resulting in no detection of the information about a heartbeat.

If a difference is taken on a time base as in the conventional art, moreover, it is necessary to take a phase difference into consideration. However, the biological information detecting device according to the invention calculates a difference in a power spectrum on a frequency axis. Therefore, it is possible to carry out an analysis in a necessary frequency region by removing a frequency component generated by the running vibration without taking the phase difference into consideration.

The biological information detecting device according to the invention calculates the difference between the power spectra of the two vibration detecting means and obtains the information about a heartbeat by taking note of the fundamental frequency region of the heartbeat in place of the frequency band of the resonant frequency of the living body. Therefore, it is possible to detect the information about the heartbeat of the living body even if a vibration noise is made on an outside.

In the biological information detecting device according to the invention, referring to a first invention, power spectra of respective output signals of first vibration detecting means for detecting a vibration of a living body and second vibration detecting means provided separately through vibration attenuating means are calculated by first calculating means, a difference between both of the power spectra thus calculated is calculated for each frequency by second calculating means, and third calculating means obtains, as an information about a heartbeat, a frequency at which the power spectra are maximized in a fundamental frequency region of a heartbeat which is preset based on a result of the calculation of the second calculating means.

The difference between the power spectra of the two vibration detecting means is calculated and the information about a heartbeat is obtained by taking note of the fundamental frequency region of the heartbeat in place of the frequency band of the resonant frequency of the living body. Therefore, it is possible to provide a biological information detecting device having a high utility which can detect the information about the heartbeat of the living body even if a vibration noise is made on an outside.

Referring to a second invention, particularly, the contact object according to the first invention is at least one of objects with which a part of a human body to be a living body can come in contact for living, for example, clothes, a bracelet, a belt, a necklace, a chair, a toilet seat, a bathtub, scales, bedclothes, a seat for a vehicle, a floor, a cell phone and a PDA. The first vibration detecting means is provided in various objects to be used in a daily life. Therefore, it is possible to obtain the information about a heartbeat everywhere at any time so that a convenience can be enhanced.

In a third invention, particularly, the first vibration detecting means, the vibration attenuating means and the second vibration detecting means according to the first invention are molded integrally like a sheet and are provided between a seat belt of a car and a living body on a seat. They are molded like the sheet. Therefore, it is possible to detect a vibration based on a heartbeat within a wide range between the seat belt and the living body on the seat. Consequently, it is possible to enhance precision in the detection of the information about the heartbeat.

In a fourth invention, particularly, the first vibration detecting means, the vibration attenuating means and the second vibration detecting means according to the first invention are molded integrally like a sheet and are provided in a back side of a seat, the first vibration detecting means is disposed in contact with a back side of the seat of a cushion portion provided in a back face of the seat, and the second vibration detecting means includes a spacer formed by an elastic member on a surface. They are molded like the sheet. Therefore, it is possible to detect a vibration within a wide range based on a heartbeat propagated from the living body on the seat to the back face side of the seat. Consequently, it is possible to enhance precision in the detection of the information about the heartbeat. Moreover, the first vibration detecting means is disposed in contact with the back side of the seat of the cushion portion provided in the back face of the seat. Therefore, a feeling of incompatibility is not given to seating on the back face side of the seat. Furthermore, the second vibration detecting means is provided with the spacer formed by an elastic member on a surface, and the running vibration is attenuated by the spacer when it is propagated to the second vibration detecting means so that an unnecessary vibration is propagated to the second vibration detecting means or the first vibration detecting means with difficulty. Consequently, the precision in the detection of the information about the heartbeat can further be enhanced.

In a fifth invention, particularly, the first vibration detecting means and the second vibration detecting means according to the third or fourth invention are formed with a cable-shaped piezoelectric sensor having a flexibility provided on a foundation cloth in a meandering form, and a straight part in a meandering portion of the piezoelectric sensor is provided in almost parallel with a vertical direction of the seat. The cable-shaped piezoelectric sensor having a flexibility is used. Therefore, the degree of freedom of a provision can be enhanced, for example, a bending provision can be carried out, and furthermore, the straight part in the meandering portion of the piezoelectric sensor is provided to be almost parallel with the vertical direction of the seat. Even if the vibration in the vertical direction to be the main vibrating component of the running vibration is received, therefore, the piezoelectric sensor is deformed with difficulty and is influenced by the running vibration with difficulty. Consequently, it is possible to enhance precision in detection.

In a sixth invention, the vibration attenuating means according to any of the first to fifth inventions has such a characteristic as to attenuate at least a vibration in a fundamental frequency region of a heartbeat. When the heartbeat vibration is propagated from the first vibration detecting means to the second vibration detecting means, the vibration attenuating means attenuates at least the vibration in the fundamental frequency region of the heartbeat. Therefore, a difference in a power spectrum between the respective output signals of the first vibration detecting means and the second vibration detecting means in the fundamental frequency region of the heartbeat can be caused to be clearer. Therefore, it is possible to further enhance the precision in the detection of the information about the heartbeat.

In a seventh invention, the first calculating means according to any of the first to sixth inventions successively calculates a moving average value within a certain time range for each of the output signals of the first vibration detecting means and the second vibration detecting means and calculates a power spectrum of time series data of the moving average value. By calculating the moving average value, it is possible to remove an unnecessary vibration component which is caused by the running vibration. Therefore, it is possible to further enhance the precision in the detection of the information about the heartbeat.

In an eighth invention, a plurality of vibration detecting means provided directly on a living body or provided on an object coming in contact with the living body and serving to detect a vibration of the living body is disposed through vibration attenuating means having a certain vibration attenuating characteristic, and frequency components of respective outputs are obtained and a frequency at which a difference is maximized in a fundamental frequency region of a heartbeat is acquired as an information about the heartbeat. The difference in the frequency component between the respective outputs of the vibration detecting means is obtained and the information about the heartbeat is acquired by taking note of the fundamental frequency region of the heartbeat. It is possible to provide a biological information detecting device having a high utility which can detect the information about the heartbeat of the living body even if a vibration noise is made on an outside.

In a ninth invention, there are provided first vibration detecting means for detecting a vibration of a living body and second vibration detecting means opposed to the first vibration detecting means and disposed through vibration attenuating means having a vibration attenuating characteristic, a frequency component of an output signal of each of the first vibration detecting means and the second vibration detecting means being obtained and a frequency at which a difference is maximized in a fundamental frequency region of a heartbeat being acquired as an information about the heartbeat. Therefore, it is possible to provide a biological information detecting device having a high utility which can detect the information about the heartbeat of the living body even if a vibration noise is made on an outside.

### <Brief Description of the Drawings>

Fig. 1 is a view showing an office support system according to a first embodiment of the invention.
Fig. 2 is a block diagram showing the structure of the system.
Fig. 3 is a block diagram showing structure of the system.
Fig. 4 is a block diagram showing the aromatherapy system of the system.
Fig. 5 is a block diagram showing the screen saver system of the system.
Fig. 6 is a view showing a palm pad in the system.
Fig. 7 is a view showing a pressure cable sensor.
Fig. 8 is an explanatory chart showing the output of the sensor.
Fig. 9 is a chart for explaining the body motion information processing operation of a processing portion.
Fig. 10 is a chart for explaining a specific example of a biological condition decision made by a deciding portion (2 elements).
Fig. 11 is a diagram for explaining a specific example of the biological condition decision made by the deciding portion (three elements).
Fig. 12 is a chart showing the output of the sensor, and an information about a heartbeat and an information about a breath which are obtained therefrom.
Fig. 13 is a flowchart showing the operation of the support system.
Fig. 14 is a view showing a biological sensor according to a second embodiment of the invention.
Fig. 15 is a view showing a bracelet according to a third embodiment of the invention.
Fig. 16 is a view showing a self-training system according to a fifth embodiment of the invention.
Fig. 17(a) is a view showing the structure of a seat device, illustrating a seventh embodiment of the biological condition deciding device according to the invention, and Fig. 17(b) is an enlarged view showing a seat belt.
Fig. 18 is a view showing another method of attaching a human body sensor to the seat belt.
Fig. 19 is a block diagram showing the function of control means.
Fig. 20 is a block diagram for explaining a function related to the heartbeat information processing operation of the processing portion.
Fig. 21 is a chart for explaining the body motion information processing operation of the processing portion.
Fig. 22 is a chart for explaining a specific example (two elements) of the biological condition decision made by the deciding portion.
Fig. 23 is a diagram for explaining a specific example (three elements) of the biological condition decision made by the deciding portion.
Fig. 24 is a chart for explaining a processing of deciding a biological condition by using a signal waveform.
Fig. 25 is a view showing the structure of a child seat, illustrating an eighth embodiment of the biological condition deciding device according to the invention.
Fig. 26 is a view showing an example of the attachment of a seat belt pad.
Fig. 27 is a view showing the structure of a sensing unit 7 in a biological information detecting device according to a ninth embodiment of the invention.
Fig. 28 is a view showing the case in which the sensing unit 6007 of the device is provided in a seat 6013 of a car, a main part being taken away.
Fig. 29 is a block diagram showing the device.
Fig. 30 is a characteristic chart representing the temporal transition of an electrocardiogram SS0 of a human body on a seat in the case in which a car applying the device thereto is set in an idling condition, an output signal SS1 of first vibration detecting means 6008, an output signal SS2 of second vibration detecting means 6010, and a difference SS3 between SS1 and SS2.
Fig. 31 is a flowchart showing the detection of a heart rate in the device.
Fig. 32 is a characteristic chart showing a difference in a power spectrum between both SS1 and SS2 calculated by second calculating means 6018 in the idling condition.
Fig. 33 is a characteristic chart representing the temporal transition of an electrocardiogram SS0 of a human body on a seat in the case in which a car applying the device thereto is set in a running condition, an output signal SS1 of first vibration detecting means 6008, an output signal SS2 of second vibration detecting means 6010, and a difference SS3 between SS1 and SS2.
Fig. 34 is a characteristic chart showing a difference in a power spectrum between both SS1 and SS2 calculated by second calculating means 6018 in the running condition.
Fig. 35 is a view showing a structure in which the sensing unit 6007 of the device is provided between a seat belt 6006 of the car and a living body on the seat.
Fig. 36(a) is a view showing the structure of a conventional biological information detecting device and Fig. 36(b) is a view showing a structure in which the conventional biological information detecting device is attached to a seat belt.

In the drawings, O denotes a person to be measured, 1 denotes a biological sensor, 100 denotes a keyboard, 200 denotes a palm rest, 210 denotes a body portion, 211 denotes a low repulsive urethane layer, 212 denotes an urethane layer, 240 denotes a bag-shaped cover, 250 denotes a support portion for a palm rest, 300 denotes a piezoelectric sensor, 320 denotes a sheet-shaped member, 321 denotes a flexible film, 322a and 322b denote a sensor portion, 330 denotes a support board, 331 denotes a dent, 332 denotes a plastic board, 360 denotes a piezoelectric cable sensor, 400 denotes storage means, 500 denotes deciding means, 600 denotes an aromatherapy system, 700 denotes a screen saver system, 5010 denotes a seat belt, 5011 denotes a human body sensor, 5020 denotes a sheet, 5021 denotes a backrest portion, 5022 denotes a seating portion, 5023 denotes a car sensor, 5030 denotes a control portion, 5301 denotes a signal input portion, 5303 denotes a processing portion, 3031 denotes first calculating means, 3032 denotes second calculating means, 3033 denotes third calculating means, 3034 denotes fourth calculating means, 3035 denotes fifth calculating means, 5305 denotes a deciding portion, 5307 denotes a driving control portion, 5040 denotes storage means, 360 denotes a piezoelectric cable sensor, 361 denotes a core (center electrode), 362 denotes a piezo element material, 363 denotes an outer electrode, 364 denotes a PVC (vinyl chloride resin), 5110 denotes a child seat belt, 5120 denotes a child seat body, 5200 denotes a seat belt for a rear seat, 5300 denotes a seat belt pad, Y denotes an output signal, X denotes a threshold, 50S0, 50S1 and 50S2 denote a signal, 50R1 and 50R2 denote a comparing signal, 6008 denotes first vibration detecting means, 6009 denotes vibration attenuating means, 6010 denotes second vibration detecting means, 6011 denotes a foundation cloth, 6012 denotes a piezoelectric sensor, 6013 denotes a seat, 6014 denotes a cushion portion, 6015 denotes a spacer, 6017 denotes a first calculating portion, 6018 denotes a second calculating portion, 6019 denotes a third calculating portion, and 6020 denotes a fourth calculating portion.

### <Best Mode for Carrying Out the Invention>

Embodiments of the invention will be described below in detail with reference to the drawings.

### (First Embodiment)

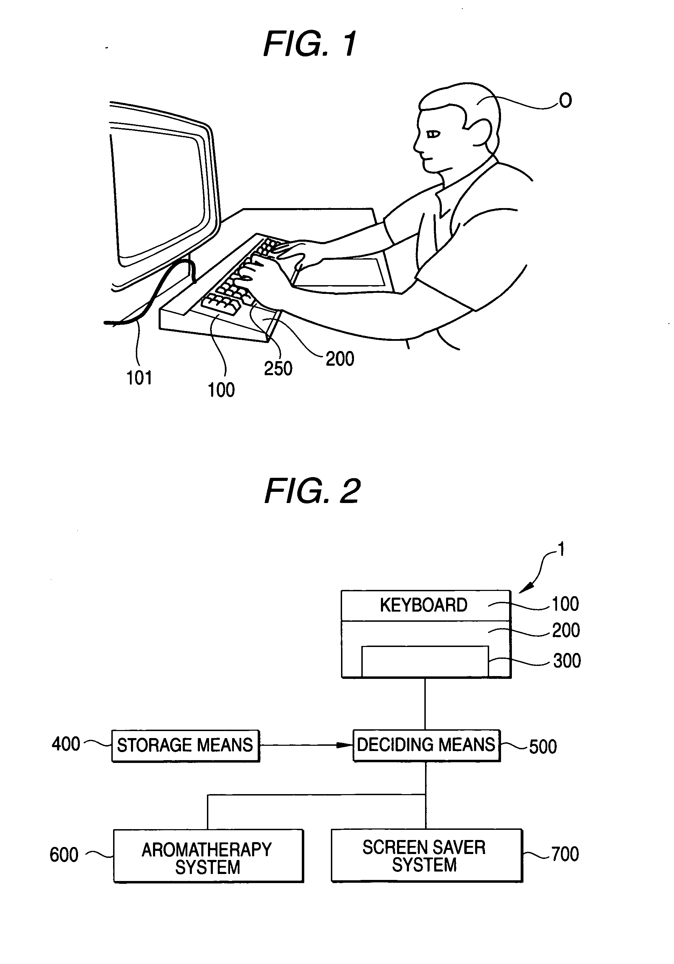

In the embodiment, an office support system will be described. As shown in Fig. 1, the office support system is attached, as acceleration detecting means, to a keyboard 100 of a personal computer, and serves to detect the condition of a living body by using a piezoelectric cable sensor (which is not shown and will be described below) provided on a palm rest 200 arranged with which at least a part of the palm of a person to be measured comes in contact. As shown in Fig. 2, a biological sensor 1 comprises a piezoelectric sensor 300, storage means 400 for storing an information about a heartbeat, an information about a body motion and an information about a breath, and deciding means 500 for deciding the condition of a living body based on the informations stored in the storage means 400. In the biological sensor 1, the information about a heartbeat, the information about a body motion and the information about a breath are extracted from the output information detected by the piezoelectric sensor 300, and reference is made to a biological information in the normal condition of the measured person which is prestored in the storage means 400 to decide the condition of the living body based on a difference from the same information. The biological information obtained by the biological sensor is objectively decided to control the driving operations of an aromatherapy system 600 and a screen saver system 700.

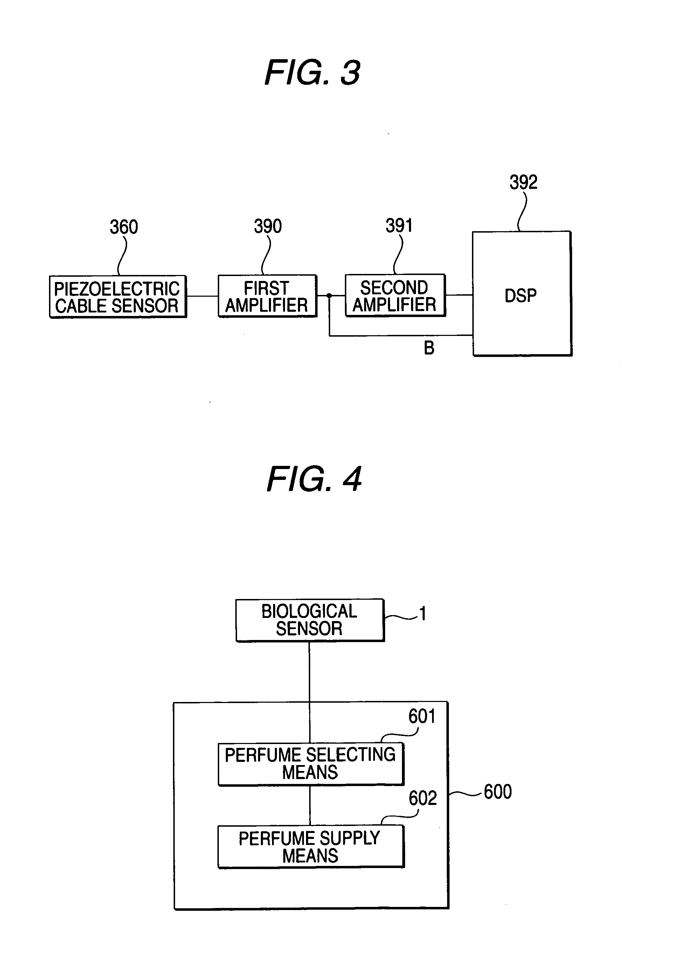

Moreover, the piezoelectric sensor 300 comprises a piezoelectric cable sensor 360, a first amplifier 390 for amplifying the output signal of the piezoelectric cable sensor 360, a second amplifier 391 for further amplifying the output of the first amplifier, and a processing portion 392 formed by a DSP (Digital Signal Processor) for processing the outputs of the first amplifier and the second amplifier as shown in the block diagram of Fig. 3. The first amplifier 390 serves to amplify the output of the piezoelectric cable sensor at approximately 100 times, and the second amplifier 391 serves to amplify the output of the first amplifier 390 at approximately 10 to 100 times.

As shown in Fig. 4, the aromatherapy system 600 comprises perfume selecting means 601 for selecting any perform to be supplied based on an information output from the biological sensor 1, and perfume supply means 602 for supplying the perfume toward a measured person O based on the result of the selection obtained by the perform selecting means 601. By this structure, in the embodiment, the biological sensor is supported to progress the thought of an input person more smoothly and efficiently by supplying a fresh scent interlockingly with the aromatherapy system.

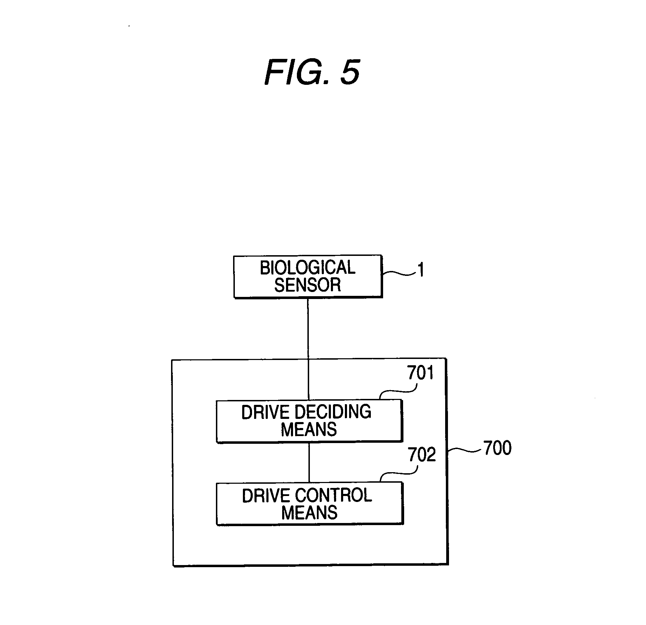

As shown in Fig. 5, the screen saver system 700 comprises drive deciding means 701 for deciding whether a screen saver is to be driven or not based on the information output from the biological sensor 1, and drive control means 702 for controlling the driving operation of the screen saver based on the result of the decision obtained by the drive deciding means 701. With this structure, by incorporating the biological sensor into the decision of the operation of the screen saver system 700, it is possible to detect, by means of the biological sensor, whether an input person thinks, leaves a seat or takes a nap when a predetermined time passes without the input person carrying out a keyboard input and the screen saver is operated. In the case in which the thinking is continuously carried out even if the time passes without the keyboard input, therefore, it is also possible to control the operation of the screen saver to leave the operation of the screen saver and to prevent the occurrence of a bad influence that the keyboard input dare to be carried out to disturb the thinking or the screen saver is operated to disturb the thinking.

At this time, moreover, it is also possible to carry out an application to a system for supplying oxygen or a sound system for giving music in addition to the control of the operation of the screen saver, and a more comfortable office environment can be created by using them together.

Next, description will be given to the palm rest including the piezoelectric cable sensor to be the biological sensor to be used herein and the piezoelectric sensor using the piezoelectric cable sensor to be utilized therefor.

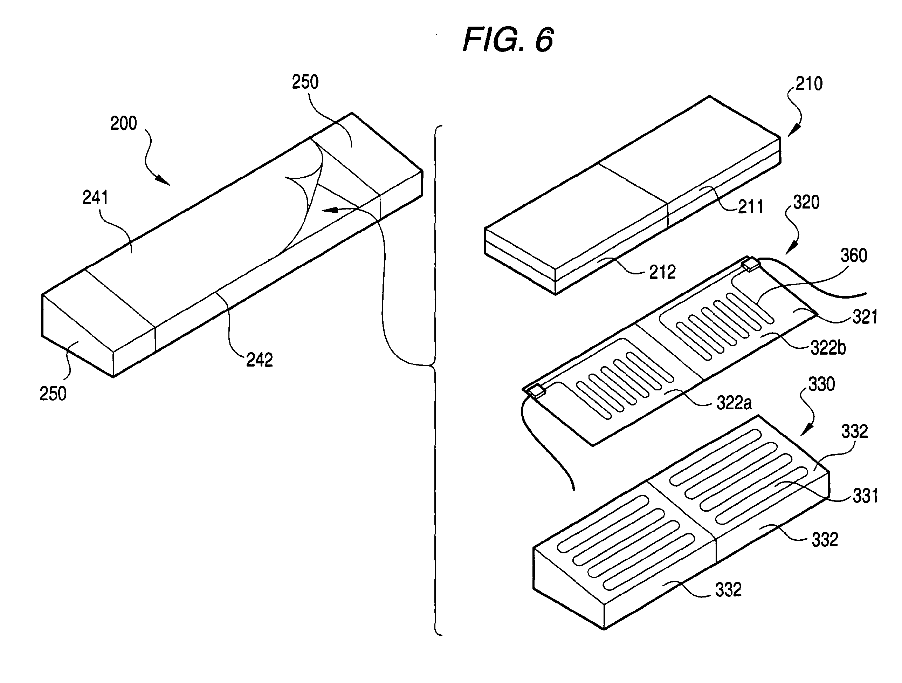

As shown in Fig. 6, a body portion 210 of the palm rest 200 including the biological sensor is constituted by a low repulsive urethane layer 211 and an ordinary urethane layer 212 provided thereunder. The thickness of the low repulsive urethane layer 211 is equal to or smaller than a half of the thickness of the whole palm rest body portion 210.

While the biological sensor 1 is constituted by the piezoelectric sensor 300, the storage means 400 and the deciding means 500 as shown in Fig. 1, the storage means 400 may be provided in the palm rest 200 and may be connected to a personal computer through a USB terminal and the deciding means 500 may be implemented in the personal computer. The piezoelectric sensor 300 is constituted by a flexible sheet-shaped member 320, a flexible film 321 and sensor portions 322a and 322b provided by meandering the piezoelectric cable sensor 360 like a wave thereon. Herein, the piezoelectric cable sensor is divided into two parts on left and right sides and can detect respective data.

A support board 330 is provided with a large number of dents 331 on the surface of a plastic board 332 for a support so that a concavo-convex surface is formed. These functions and advantages will be described below.

240 denotes a bag-shaped cover formed by folding a cloth 241 double and causing three sides to be openable by means of a fastener 242. When the fastener 242 is opened, and the palm rest body portion 210, the piezoelectric sensor 300 and the plastic board 332 for a support are accommodated in the bag-shaped cover 240 in a superposition state from a top in order and the faster 242 is closed, the palm rest is obtained.

250 denotes a frame for a palm rest support which is attached to the keyboard 100, and the palm rest is fixed to the keyboard with an interposition between both sides of the palm rest.

Next, description will be given to the "low repulsive urethane" constituting the palm rest body portion 210 and "ordinary urethane".

The "low repulsive urethane" is constituted by selecting the composition of an urethane foam, that is, the type of polyisocyanate and the number of functional groups and hydroxyl group value of polyol and prescribing them in order to cause a glass transition at a temperature at which the urethane foam is used (usually, a room temperature), and giving a low repulsion by the glass transition phenomenon.

On the other hand, the "ordinary urethane" is prescribed in such a manner that the glass transition is not caused at the temperature at which the urethane foam is used (the room temperature), and therefore, it has a high repulsion.

Both physical property values are as follows.

The ordinary urethane foam is divided into three types depending on a hardness.
There are soft, medium and hard types.
1) An urethane foam of the soft type has a density of 20±2 kg/m³, a hardness of 6±1.5, a tear strength of 0.2 kg/cm or more, a tensile strength of 0.6 kg/cm² or more, an extension rate of 150% or more, a repulsive elasticity of 35% or more, and a residual strain of 6% or less.
2) An urethane foam of the medium type has a density of 20±2 kg/m³, a hardness of 11±1.5, a tear strength of 0.2 kg/cm or more, a tensile strength of 0.7 kg/cm² or more, an extension rate of 120% or more, a repulsive elasticity of 35% or more, and a residual strain of 6% or less.
3) An urethane foam of the hard type has a density of 21±2 kg/m³, a hardness of 15±2.0, a tear strength of 0.2 kg/cm or more, a tensile strength of 0.7 kg/cm² or more, an extension rate of 120% or more, a repulsive elasticity of 35% or more, and a residual strain of 6% or less.

Moreover, an urethane foam having a low repulsion has a density of 65±10 kg/m³, a hardness of 5.5±2.0, a tear strength of 0.2 kg/cm or more, a tensile strength of 0.5 kg/cm² or more, an extension rate of 150% or more, a repulsive elasticity of 5% or less, and a residual strain of 3% or less.

The density and the hardness are measured according to JIS-K6401, the tear strength, the tensile strength and the extension rate are measured according to TIS-K6301, and the repulsion elasticity and the residual strain are measured according to JIS-K6401.

While the palm rest body portion 210 may be constituted by only a low repulsive urethane layer, moreover, the reason why it has a two-layer structure including the low repulsive urethane layer 211 and the ordinary urethane layer 212 is that it becomes expensive and sinks excessively if a sufficient thickness is to be obtained by only the low repulsive urethane layer, which is not preferable.

In this case, apparently, it is preferable that a portion which is a little less than a half of a whole thickness should be constituted by the low repulsive urethane layer and an urethane layer to be an ordinary cushion material should be stuck to a lower part of the low repulsive urethane layer.

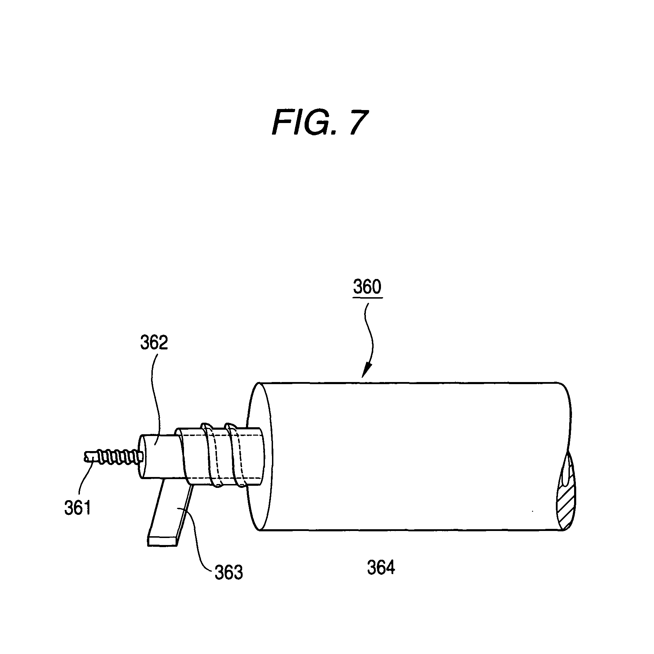

The piezoelectric cable sensor to be used herein is a cable-shaped sensor using a piezo element material as shown in Fig. 7. The piezoelectric cable sensor 360 comprises a core (center electrode) 361 on a center in an axial direction, a piezo element material 362 provided to cover the periphery of the center electrode 361, and furthermore, an outer electrode 363 provided around the piezo element material 362, and is constituted by covering an outermost periphery with a flexible resin such as a PVC (polyvinyl chloride) 364.

The piezoelectric cable sensor 360 uses, for the piezo element material 362, a resin type material having a heat resistance which has a maximum allowable temperature of 120 □ and has been originally developed by the applicant, and can be used in a temperature region (1200 or less) which is higher than 900 to be the maximum allowable temperature of a conventionally typical polymer piezo element material (uniaxial stretched polyvinylidene fluoride) or piezo element material (a piezo element material of chloprene and piezoelectric ceramic powder). The piezo element material 362 is constituted by a resin having a flexibility and piezoelectric ceramic, and furthermore, is formed by using a flexible electrode including a coil-shaped metal center electrode and a film-shaped outer electrode and has a flexibility which is equivalent to that of an ordinary vinyl cord.

In addition, the piezoelectric cable sensor 360 has a high sensitivity which is almost equal to that of a polymer piezo element material and has a high sensitivity which is equivalent to that of the polymer piezo element material in such a low frequency region (10 Hz or less) as to detect the interposition of a human body. The reason is that the relative dielectric constant (approximately 55) of the piezo element material 362 is greater than that (approximately 10) of the polymer piezo element material, and therefore, a reduction in the sensitivity is small also in the low frequency region (10 Hz or less). In particular, recently, it has been apparent that a high sensitivity can also be obtained at several Hz. A detection can also be carried out in a fundamental frequency band in addition to a resonant frequency band and a measurement can easily be performed.

The piezo element material 362 is constituted by the complex of a resin type material and piezoelectric ceramic powder having a particle size of 10µm or less, and a vibration detecting characteristic is implemented by the composition and particle size of the ceramic powder and a flexibility is implemented by the selection of the resin material. The piezo element material 362 uses chlorine type polyethylene as the resin type material to implement a high heat resistance (1200) and a flexibility giving an easy formation, and furthermore, can be formed in a simple manufacturing process in which crosslinking is not required.

The piezoelectric cable sensor 360 thus obtained can give a piezoelectric performance by forming the piezo element material 362 and then applying a high DC voltage of several kV/mm to the piezo element material 362, thereby carrying out a polarization over the piezo element material 362. In the case in which a minute defect such as a crack is present in the piezo element material 362, the defect portion is easily discharged so that both electrodes are apt to be short-circuited. For this reason, a sufficient polarization voltage cannot be applied. By establishing an original polarizing process using an auxiliary electrode constituted to adhere to the piezo element material 362 having a constant length in the invention, however, it is possible to detect and avoid the defect and to stabilize the polarization, thereby increasing a length to be several tens m or more.

In the piezoelectric cable sensor, moreover, a coil-shaped metal center electrode is used for the center electrode 361 and a film-shaped electrode (a three-layer laminate film including aluminum - polyethylene terephthalate - aluminum) is used for the outer electrode 363. Consequently, the adhesion of the electrode to the piezo element material 362 can be maintained, and furthermore, the connection of an external lead wire can easily be carried out and a flexible cable-shaped mounting structure can be obtained. The center electrode 361 is a copper - silver alloy coil, the outer electrode 363 is a three-layer laminate film including aluminum - polyethylene terephthalate - aluminum, the piezo element material 362 is a polyethylene type resin + piezoelectric ceramic powder, and an envelope is a thermoplastic. Consequently, a relative dielectric constant is 55, an amount of generation of electric charges is 10 to 13 C (coulomb)/gf, and a maximum allowable temperature is 120□.

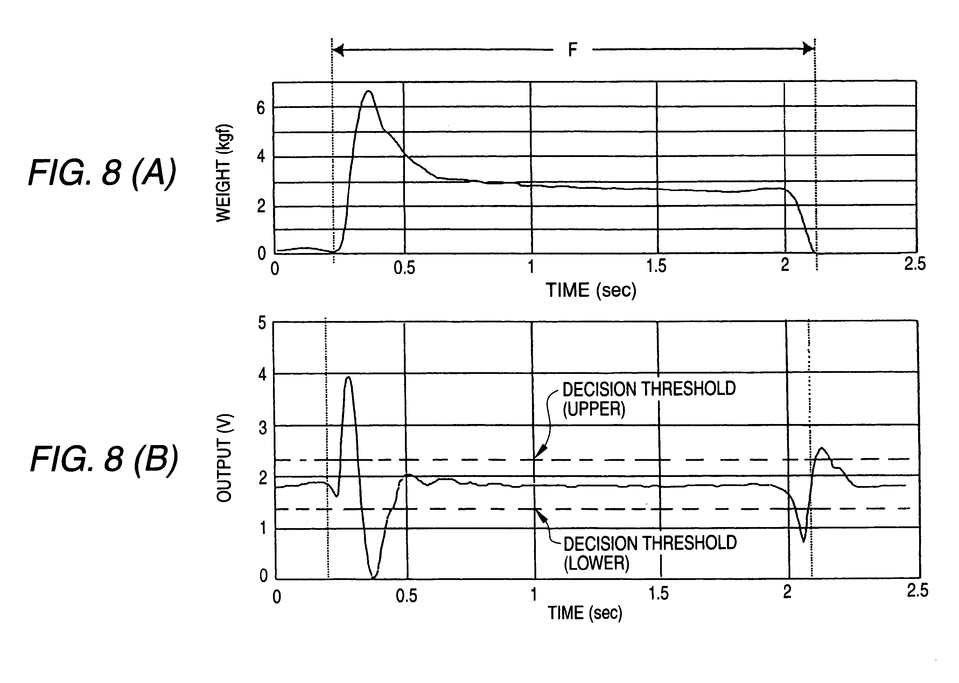

Fig. 8 is a chart showing a load applied to the piezoelectric cable sensor 360 and a sensor output characteristic. The applicant carried out an experiment for a relationship between the load of the piezoelectric cable sensor 360 and a sensor output. As a result, the sensor output presents a phenomenon in (b) when a bending load in (a) is applied to the piezoelectric cable sensor 360.
(1) More specifically, when the load is not applied to the piezoelectric cable sensor 360 at a time t0, the sensor output indicates 2(V).
(2) When a bending load is applied in a constant direction to the piezoelectric cable sensor 60 at a time t 1, the sensor output is increased to be 4(V) the moment that the bending load is applied, and is inversed immediately thereafter to be 0(V) and subsequently returns to 2(V) again.
(3) Then, the sensor output maintains to indicate 2(V) with bending.
(4) When the piezoelectric cable sensor 60 is recovered to an original state at a time t3, the sensor output is decreased to be 0.8(V) at that moment and is immediately inverted to be 2.2(V), and thereafter returns to 2(V) again.

Thus, the piezoelectric cable sensor generates an output reacting to an acceleration. Only the moment that a force is applied, a signal is output. Even if the force is then applied continuously, the output is zero until a fluctuation is caused, that is, in the case in which an acceleration is zero. There is a characteristic in which the output is given only the moment that the force is removed. In the case in which the piezoelectric cable sensor is laid with bending like a wave in the lower part of the palm rest, accordingly, an output is generated only when a force is applied to a part of the piezoelectric cable sensor, that is, a change in a pressure is caused.

Herein, the piezoelectric cable sensor is provided on a concavo-convex plate in order to increase the detection sensitivity of the piezoelectric cable sensor. When a portion facing a concave portion in the piezoelectric cable sensor is pressed from above, a pressurization is selectively carried out in a downward direction. Therefore, only a change appears as a signal so that a large signal can be obtained.

Moreover, the piezoelectric sensor may be laid over the lower surface of a sandwich-shaped bed pad in which an ordinary cushion layer formed of urethane is provided as a palm pad on the upper and lower parts of a low repulsive urethane layer.

Description will be given to the operation of a (personal computer) input support system using the biological sensor.

Description will be given to the case in which an input person (a person to be measured) carries out an input operation by using the palm rest as shown in Fig. 1.

First of all, the sensor portions 322a and 322b formed with the piezoelectric cable sensor 360 meandering detect an acceleration transmitted through the palm rest body portion 210 abutting on a palm. Herein, data on both left and right hands can be fetched.

The output signals of the sensor portions 322a and 322b corresponding to the right and left hand sides are set to be the output signal of the piezoelectric cable sensor 360. In the processing portion 392, each of vibration signals output therefrom is analog-to-digital converted into a digital value, and a moving average value is then calculated successively within a certain time range and the power spectrum of the time series data of the moving average value is calculated.

Thereafter, the sum of both of the power spectra is calculated for each frequency. Based on the result of the calculation, subsequently, a frequency at which the power spectrum is maximized is obtained as an information about a heartbeat in the fundamental frequency region of the heartbeat which is preset. The frequency thus obtained is multiplied by 60 to acquire a heart rate. Thus, the processing portion 392 can obtain the heart rate (the information about a heartbeat) of a driver. In the embodiment, the fundamental frequency region of the heartbeat is set to be approximately 1 to 2 Hz. In this procedure, the heart rate is calculated every second to every ten seconds.

By using the heart rate obtained in the method described above, moreover, a variation in the heart rate every second to every ten seconds is obtained. First of all, an average value and a standard deviation of the heart rate are calculated from time series data on the heart rate every second to every ten seconds, and the standard deviation of the heart rate is divided by the average value of the heart rate to obtain the variation in the heat rate. For the information about the heart rate, both the heart rate and the variation in the heart rate can be used.

Herein, the processing portion is provided in a special IC (not shown).

While the description has been given to the method in which the processing portion 392 carries out the frequency analysis over the output signals sent from the two left and right sensor portions, thereby obtaining the information about the heartbeat, the information about the heartbeat may be acquired by a method using CVRR, LF/HF or a chaos index, for example.

While the processing portion 392 obtains the information about the heartbeat by taking note of the frequency band of approximately 1 to 2 Hz in the above description, moreover, it is possible to obtain an information about a breath by taking note of a frequency band of 1 Hz or less in the same manner.

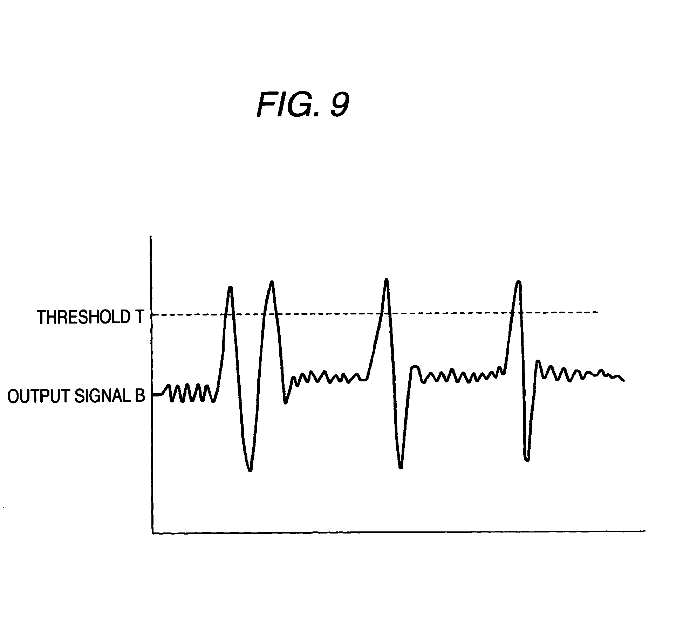

Fig. 9 is a chart for explaining a body motion information processing operation. In the processing portion 392, there is obtained the number of times that a signal (an output signal B) acquired by amplifying the output signals of the sensor portions 322a and 322b corresponding to the right and left hand sides at approximately 100 times by means of the first amplifier 390 exceeds a predetermined threshold T per unit time. The output signal B includes a vibration signal caused by a vibration generated by an external cause in addition to a vibration signal caused by the biological activity (a heartbeat or a breath) of the measured person. Since the body motion has a greater amplitude than these vibrations, however, an information about a body motion can be obtained based on only the output signal of the piezoelectric cable sensor 360.

The deciding means 500 shown in Fig. 2 decides the biological condition of the measured person by referring to biological informations (an information about a heartbeat, an information about a breath and an information about a body motion) in a usual condition of the measured person which are stored in the storage means 400 based on the result of the processing of the processing portion 303 in the piezoelectric sensor 300.

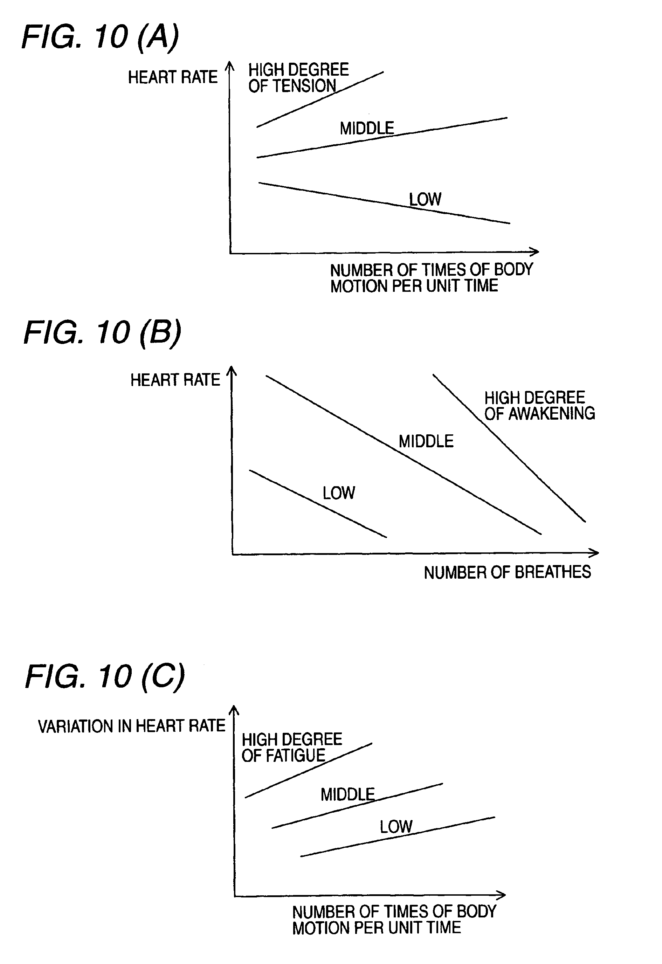

Fig. 10 is a chart for explaining a specific example of the decision of the biological condition which is made by the deciding means 500. Fig. 10(a) shows a correlation between the number of times of a body motion per unit time and a heart rate. The deciding means 500 decides a degree of tension based on the correlation. Fig. 8(b) shows a correlation between the number of breathes and the heart rate. The deciding means 500 decides a degree of awakening based on the correlation. Fig. 8(c) shows a correlation between the number of times of a body motion per unit time and a variation in the heart rate. The deciding means 500 decides a degree of fatigue based on the correlation. The correlations shown in Fig. 10 are based on the result of a measurement which is obtained by previously carrying out an experiment for the measured person, and it is assumed that these data are stored in the storage means 400. For example, an accurate degree of awakening is measured from brain waves by an experiment, and at the same time, data on the heartbeat, the breath and the body motion are collected to obtain a relationship among the degree of awakening and a heart rate, the number of breathes and a body motion pattern. At this time, it is desirable that a suitable relationship to all tastes should be obtained by permitting some freedom to a person to be inspected based on a large number of data.

In the decision of a degree of tension, as shown in Fig. 10(a), the degree of tension is decided in three stages of high, middle and low depending on any of tension degree curves obtained previously which is closer. The deciding means 500 compares the result of the processing of the processing portion 392 (an information about a heartbeat and an information about a body motion) with that in this drawing and decides the degree of tension. For example, in the case in which a heart rate is high, it can be decided that the degree of tension is high if the number of times of the body motion is small and the degree of tension is middle if the number of times of the body motion is large.

In the decision of a degree of awakening, as shown in Fig. 10(b), the degree of awakening is decided in three stages of high, middle and low depending on any of awakening degree curves obtained previously which is closer. For example, in the case in which the heart rate is high, it can be decided that the degree of awakening is middle if the number of breathes is small and the degree of awakening is high if the number of breathes is large. The deciding means 500 compares the result of the processing of the processing portion 392 (the information about the heartbeat and the information about the breath) with that in this drawing and decides the degree of tension.

In the decision of a degree of fatigue, as shown in Fig. 10(c), the degree of fatigue is decided in three stages of high, middle and low depending on any of fatigue degree curves obtained previously which is closer. Referring to Fig. 10(c), in the case in which a variation in the heart rate is almost middle, for example, it can be decided that the degree of fatigue is high if the number of times of the body motion is small and the degree of fatigue is low if the number of times of the body motion is large. The deciding means 500 compares the result of the processing of the processing portion 392 (a variation in the heart rate and the number of times of the body motion) with that in this drawing and decides the degree of fatigue.

Although the deciding means 500 decides the degree of tension, the degree of awakening and the degree of fatigue in the three stages in the above description, it is also possible to make a decision in more stages such as five stages or ten stages if the correlation is previously obtained by an experiment. It is also possible to decide the biological condition depending on a correlation between the variation in the heart rate and the number of breathes in addition to the correlation between the two elements (the heart rate and the number of times of the body motion).

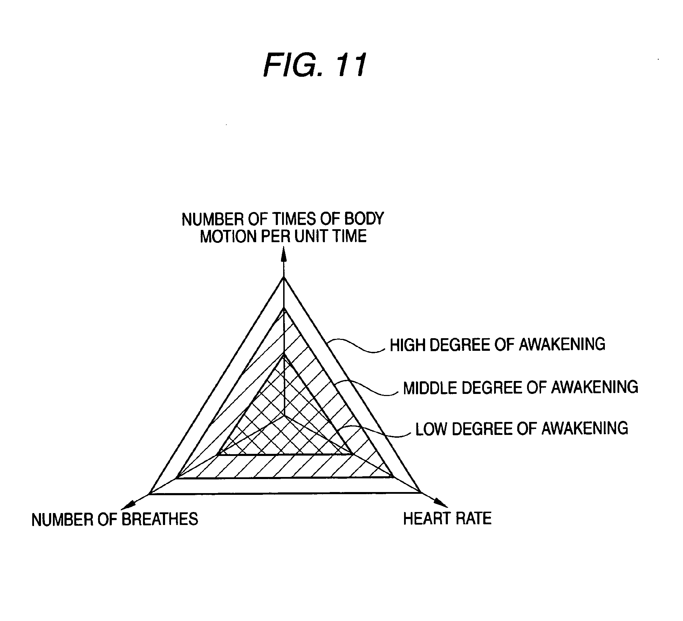

Moreover, it is also possible to obtain a correlation among three elements of the heart rate, the number of times of the body motion and the number of breathes including the number of breathes in addition to the two elements as shown in an example of Fig. 11 and to decide the degree of awakening. Consequently, it is possible to make a decision more accurately.

By adding an information about a body temperature to the correlation between the two elements or the three elements, furthermore, it is possible to make a decision with higher precision. It is possible to easily measure the body temperature by providing a body temperature sensor in a portion of the palm rest with which a wrist comes in contact in the same manner. Based on a body temperature rhythm (a circadian rhythm), moreover, it is also possible to correct the awakening degree curve.

While the description has been given to the processing of obtaining the information about the heartbeat, the information about the breath and the information about the body motion by the frequency analysis in the processing portion 392, it is also possible to make a decision based on a signal waveform.

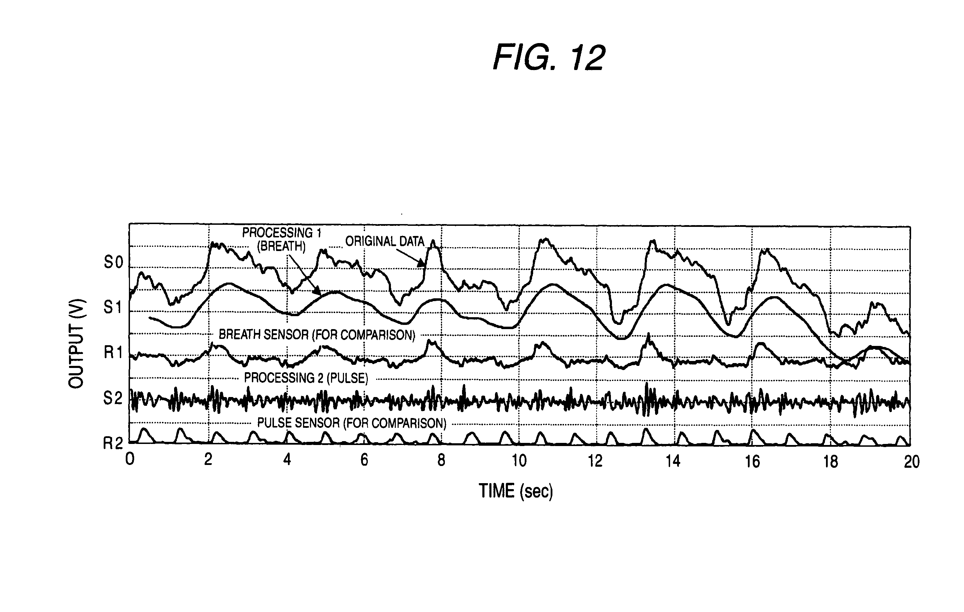

The added value of the output signal data of the left and right sensor portions 322a and 322b represents an output signal S0 shown in Fig. 12.

The output signal data divide the palm rest into two parts and a pressure sensor is provided respectively, and an output signal is obtained by the addition of left and right parts. Therefore, errors can be reduced.

Then, the output signal is fetched through a band-pass filter of 0.1 to 1 Hz and a result thus obtained is indicated as S 1 (processing 1) (which corresponds to a use for a breath).

On the other hand, the output signal is fetched through a band-pass filter of 4 to 7 Hz and a result thus obtained is indicated as S2 (processing 2) (which corresponds to a use for a heartbeat).

They are compared with signal values for a comparison which are previously measured and stored in the storage means (for example, a signal for a breath is indicated as R1 and a signal for a pulse is indicated as R2), respectively. From the result of the comparison, the deciding means 500 decides a condition for each item in consideration of both the information about the heartbeat and the information about the breath. First of all, it is decided whether an awakening condition is set or not, for example. The signal data for the comparison in various cases are stored in consideration of a normal condition, a concentrating condition and a fatigue condition, and they are compared with each other.

Based on the result of the comparison, it is decided whether or not each countermeasure operation is to be carried out corresponding to the condition of the living body and respective countermeasures are taken.

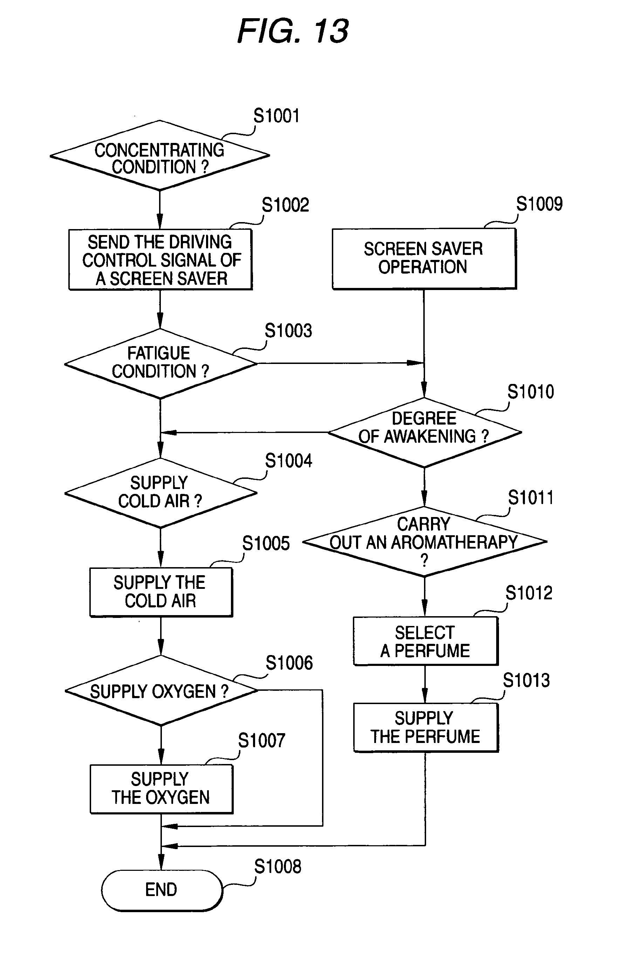

A countermeasure operation using an aromatherapy system and a screen saver system will be described with reference to a flowchart of Fig. 13.

First of all, a concentrating condition (whether a concentration is carried out or not) is decided from the output information of the biological sensor by the deciding means (Step 1001).

If it is decided that the concentration is carried out, the driving inhibit signal of the screen saver is sent (Step 1002).

Thus, the driving operation of the screen saver is inhibited. Even if the set time of the screen saver passes without a keyboard input, therefore, the concentrating condition is not disturbed but thinking can be progressed.

Next, the deciding means decides the fatigue condition (Step 1003). If it is decided that the fatigue condition is set, a healing step is started. As a healing measure, for example, it is decided whether a cold wind is supplied or not (Step 1004). If it is decided that the cold wind is supplied, the cold wind is supplied from a cold wind supply portion attached to the vicinity of a person to be measured (Step 1005).

As a next healing measure, for example, it is decided whether oxygen is supplied or not (Step 1006). If it is decided that the oxygen is supplied, the oxygen is supplied from an oxygen supply portion attached to the vicinity of the measured person (Step 1007) and the processing step is ended (Step 1008).

If it is decided that the concentration is not carried out at the deciding step S1001, subsequently, the screen saver is operated (Step 1009).

Next, the deciding means decides a degree of awakening (Step 1010). It is decided that the awakening condition is set, it is decided whether or not an aromatherapy is carried out as the healing measure (Step 1011).

If it is decided that the aromatherapy is carried out, a perfume is selected by the perfume selecting means in Fig. 4 (Step 1012) and a desirable scent is supplied from the perfume supply means attached to the vicinity of the measured person (Step 1013). Then, the processing step is ended (Step 1008).

On the other hand, if it is decided that the awakening condition is not set but a nap condition is set at the awakening degree deciding step 1010, the processing proceeds to the Step 1004 in which it is decided whether the cold wind is supplied or not.

Thus, the operation of the screen saver and the aromatherapy are controlled based on the output of the biological sensor. Therefore, it is possible to enhance the working efficiency of an operator. Moreover, the degree of fatigue is detected to take a countermeasure for inhibiting the storage of the fatigue of the operator. Consequently, an office environment can be enhanced and a comfortable working environment for people can be produced.

While the aromatherapy is used as the healing measure in the embodiment, it is also possible to use a sound system. For the biological information, moreover, it is also possible to detect the degree of tension and to select and supply proper music depending on the degree of tension.

Furthermore, a manager can take out the situation of each operator from the output of the biological sensor, and can take a measure for improving the working environment, for example, can introduce the aromatherapy or supply music in advance for the time period by summing up data to detect a time period for which many people are tired or apt to take a nap, or taking a rest.

While the biological condition (a state of mind) is decided by comparing the output signal with data in a usual condition of the measured person himself (herself) and carrying out an analysis in the embodiment, this is not restricted but it is also possible to use a method of counting the number of times that a predetermined threshold or more is obtained, thereby counting a heart rate and the number of breathes or another well-known method, for example, a method of analog-to-digital converting the output signal of the biological sensor into digital data through a microcomputer and calculating an autocorrelation coefficient from the time series data of the digital data, thereby calculating the heart rate and the number of breathes.

While the piezoelectric cable sensor is used as the biological sensor, moreover, it is also possible to use another sensor capable of detecting a vibration, for example, a coaxial electrostatic capacity sensor or a cable-shaped sensor of a pressure sensitive resistance type. If the sensor can be provided on the lower surface of the bed pad to detect the vibration through the heartbeat or breath of a human body, a band-shaped or sheet-shaped pressure sensitive sensor may be used in addition to the cable-shaped sensor.

By obtaining, as the information about the heartbeat, a frequency at which a difference between the power spectra in the fundamental frequency region of the heartbeat is maximized, furthermore, it is possible to extract the vibration signal component of the fundamental frequency region of the heartbeat appearing at approximately 1 to 2 Hz without burying the minute vibration of a human body to be a living body generated by the heartbeat in the great vibration of the human body which is caused by the vibration generated by an external cause also in the case in which the vibration is applied to the human body, for example.

The information about the heartbeat is obtained by taking note of the fundamental frequency region of the heartbeat usually appearing at approximately 1 to 2 Hz in place of the frequency band of the resonant frequency of the living body (approximately 4 to 7 Hz) differently from the conventional art. Differently from the conventional art, therefore, it is possible to prevent a heartbeat signal from being buried in the great vibration signal of the human body which is generated by the external cause, resulting in no detection of the information about the heartbeat.

Although a phase difference is to be considered if a difference is taken on a time base as in the conventional art, moreover, it is possible to remove the frequency component of a vibration which is generated by the external cause without taking the phase difference into consideration, thereby carrying out an analysis in a necessary frequency region if the difference between the power spectra is calculated on a frequency axis.

### (Second Embodiment)

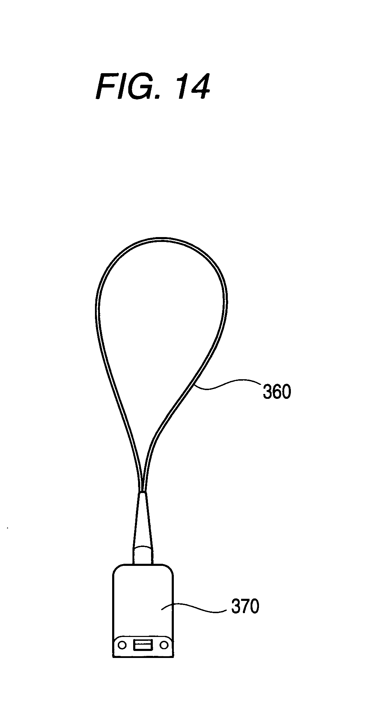

While the explanation has been given to the example in which the palm pad is incorporated as the biological sensor in the embodiment described above, it is also possible to use, as a single sensor unit, a detecting unit 370 attached to both ends of a piezoelectric cable 360 having a predetermined length. The detecting unit is formed to be connectable through a USB terminal 380 to a display portion such as a display of a personal computer as shown in Fig. 14. Since a piezoelectric cable itself is formed in the same manner as that in Fig. 7, description will be omitted.

According to this structure, an information about a living body such as an information about a heartbeat can be surely detected for a person to be measured without a feeling of incompatibility, and furthermore, a conveyance can be easily carried out, a weight is small and handling can be readily performed. For example, the unit can also be hung around the neck of the measured person so as to be used as a pendant. Moreover, it is also possible to fetch, by wireless, the output of a piezoelectric cable sensor provided on a pendant head from a personal computer. Furthermore, the piezoelectric cable sensor may be attached to the head portion of the pendant.

### (Third Embodiment)

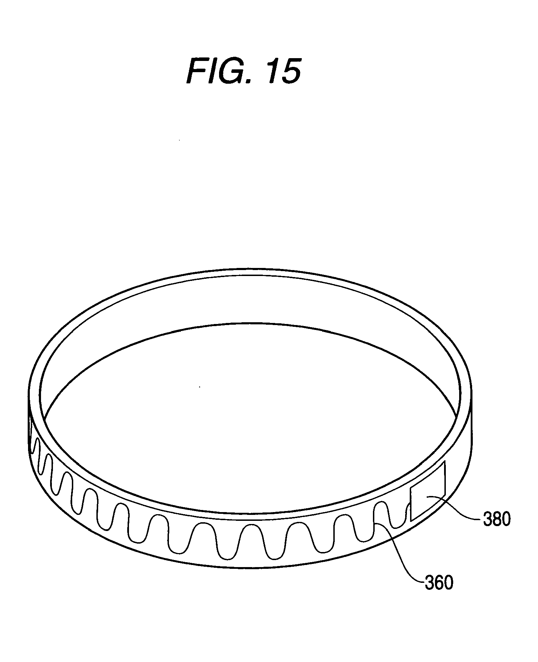

Moreover, it is also possible to bury a piezoelectric cable sensor in a bracelet and to implement the exchange of data with a personal computer by wireless through an antenna provided in a detecting unit 380 comprising the antenna as shown in Fig. 15.

Consequently, it is possible to implement a control having a high reliability without regulating the action of a person to be measured. In addition, it is possible to reliably detect an information about a living body such as an information about a heartbeat. A ring-shaped member may be incorporated in a belt attached to the waist part of the measured person in addition to the bracelet to be attached to the arm of the measured person.

### (Fourth Embodiment)

In a biological sensor, moreover, a piezoelectric cable sensor may be provided on a toilet seat in a bathroom and the output of deciding means may be displayed on a display device to objectively detect the condition of a person to be measured as a living body from informations about the living body such as a heartbeat, a body motion and a breath of the aged or a sick person to be the measured person.

Consequently, a person practicing medicine or family can reliably decide a situation and can make a reliable and objective decision without depending on a representation such as a language or a facial expression. Consequently, it is possible to implement a proper service more reliably. For example, the aged or sick people often express a different intension from a fact due to a modesty or a prejudice. Also in the case in which they say "no desire to urinate" for the question of a nursing person (a person for giving a service) due to a feeling such as a modesty or a shame even if they have the desire to urinate, for example, the nursing person can accurately grasp, from a display device, the condition of a living body based on the objective biological information and can implement an optimum and sufficient service.

### (Fifth Embodiment)

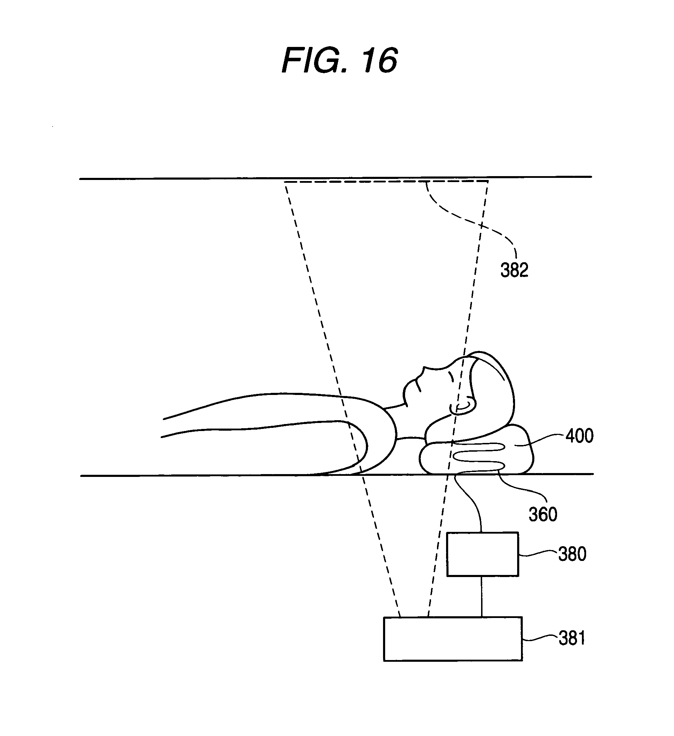

Next, an autonomic nerve training support system will be described. Herein, a piezoelectric cable sensor 360 is buried in a pillow 400 formed by low repulsive urethane as shown in Fig. 16 and at least two of a heartbeat, a body motion and a breath are taken out of an output to detect an autonomic nerve condition. In the same manner as that in the third embodiment, the output of the piezoelectric cable sensor 360 is sent to a detecting unit 380 and is displayed as character information 382 on a ceiling by a projection type display device 381.

Consequently, the trainee carries out the autonomic nerve training while seeing the information displayed on the ceiling.

By this structure, it is possible to detect the autonomic nerve condition of a living body based on the objective biological information. Therefore, it is possible to promote the consciousness of the trainee and to implement a more efficient training. For example, if the trainee to carry out a vigilance conquest training can recognize that he (she) is present in a close condition to a sleep as a biological information, a sense of security can be given to him (her) and more efficient training results can be obtained.

The biological sensor is not restricted to the pillow but may be attached to a bed pad or a surface on the human body side (inside) of a futon covering the upper part of a body.

Moreover, the display means is not restricted to a character information but the information may be displayed based on a simple image information about a twilight or a starry sky on a ceiling or the color of a light may be changed by a light representation, or an information may be displayed based on an information about music by using acoustic means, and it is desirable to use a method capable of causing the trainee to carry out a recognition comfortably and making him (her) drowsy.

### (Sixth Embodiment)

While the biological information is detected by the acceleration detecting means attached to a living body directly or through a medium in a solid state in the embodiments described above, description will be given to a method of detecting the biological information through a liquid in the embodiment.

More specifically, in the embodiment, an acceleration sensor is provided in a floating state in a bathtub to detect a vibration through water (a liquid), thereby detecting the biological information.

By objectively detecting the biological information at time of bathing, consequently, it is possible to carry out a connection to a sound system to supply an information about music or to perform an aromatherapy interlockingly with an aromatherapy system, thereby supporting a comfortable bathing time.

In addition, it is also possible to employ a utilizing method of objectively detecting the biological information at the bathing, thereby informing a nursing person or family of a sickness to prevent an accident during the bathing.

### (Seventh Embodiment)

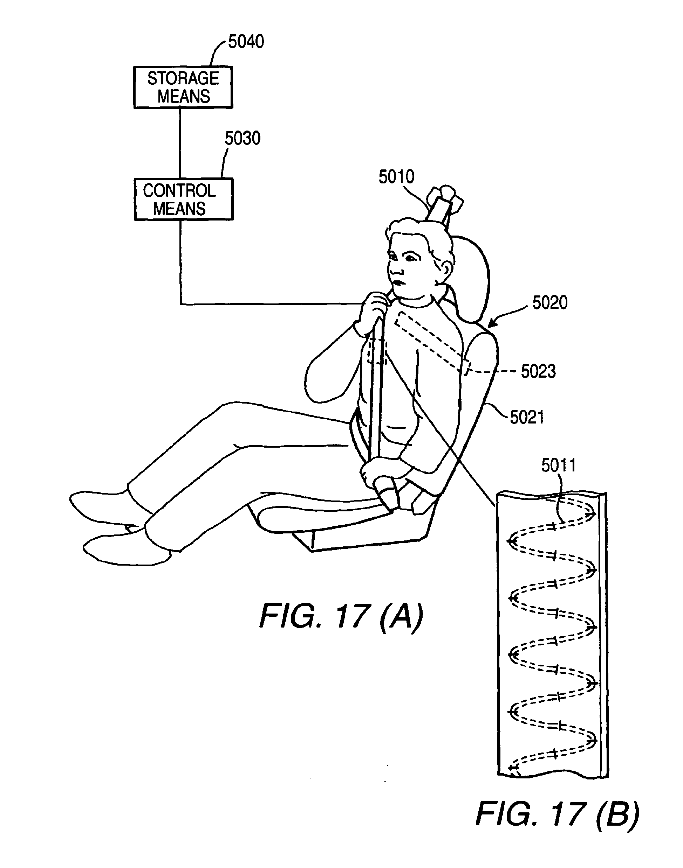

Fig. 17 is a view showing the structure of a seat device, illustrating a seventh embodiment of the biological condition deciding device according to the invention. In the embodiment, description will be given to the case in which a biological information about a driver for a car (an information about a heartbeat, an information about a breath and an information about a body motion) is detected to decide a biological condition.

As shown in Fig. 17(a), the seat device according to the embodiment comprises a seat 5010 including a backrest portion 5021 and a seating portion 5022 and having a car sensor 5023 provided on the back face of the backrest portion 5021, and a seat belt 5010 formed integrally with a human body sensor 5011, and they are connected to control means 5030 and storage means 5040 for storing informations output from the human body sensor 5011 and the car sensor 5023. The human body sensor 5011 serves to detect at least two of an information about a heartbeat, an information about a body motion and an information about a breath of a driver, and the car sensor 5023 serves to detect an information about a vibration of a vehicle.

A cable-shaped sensor (a piezoelectric cable sensor) using a piezo element material developed practically by the applicant is used for the human body sensor 5011 and the car sensor 5023.

The human body sensor 5011 is sewn in a meandering (bending) state on the face side of the seat belt 5010 which comes in contact with the human body as shown in Fig. 17(b). Consequently, a vibration signal can be received within a wide range, and furthermore, a stress is easily applied to the bending portion. Therefore, the amount of extension or contraction is increased with respect to the amount of displacement. In other words, the human body sensor 5010 is integrated with the seat belt 5010 in the meandering state so that the sensitivity of the human body sensor 5010 is increased.

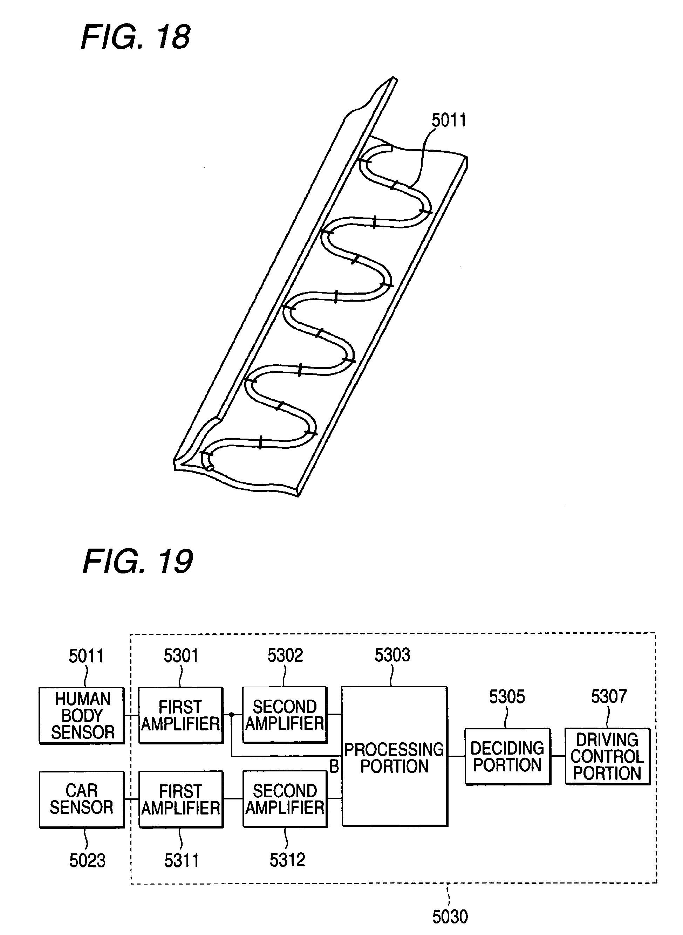

As shown in Fig. 18, the human body sensor 5011 may be integrated with the seat belt by sewing a piezoelectric cable sensor onto a half face on either side of a cloth for a belt which has an almost double of the width of a finished seat belt, folding the cloth with the sensor placed on an inside and sewing and finishing the end (outer edge portion) of the cloth. By sewing the human body sensor 5011 onto the inner part of the seat belt, it is possible to attach the seat belt with a smaller feeling of incompatibility.

Moreover, the car sensor 5023 is sewn onto the back face side of the backrest portion 5021 in a meandering state. The car sensor 5023 may be provided in any of positions placed apart from a driver, for example, the lower part of the seating portion 5022, the inner part of the seating portion 5022 or the backrest portion 5021, or a vehicle body (a ceiling or a steering wheel in the vehicle). It is desirable that the position should be placed apart from the driver in a portion other than the upper part of the seat belt.

Fig. 19 is a block diagram showing the function of the control means 5030. As shown in Fig. 19, the control means 5030 comprises first amplifiers 5301 and 5311 for amplifying the strengths of vibration signals (biological informations) output from the human body sensor 5011 and the car sensor 5023 at approximately 100 times respectively, second amplifiers 5302 and 5312 for amplifying the output signals of the first amplifiers 5301 and 5311 at approximately 10 to 100 times respectively, a processing portion 5303 for processing the cutput signals of the second amplifier 5302 and 5312 and the first amplifier 5301, a deciding portion 5305 for deciding the biological condition of a driver based on the vibration signals output from the car sensor 5023 and the human body sensor 5011, and a driving control portion 5307 for controlling a driving operation in such a manner that each portion of a music supply device carries out a predetermined operation based on the result of the decision obtained by the deciding portion 5305.

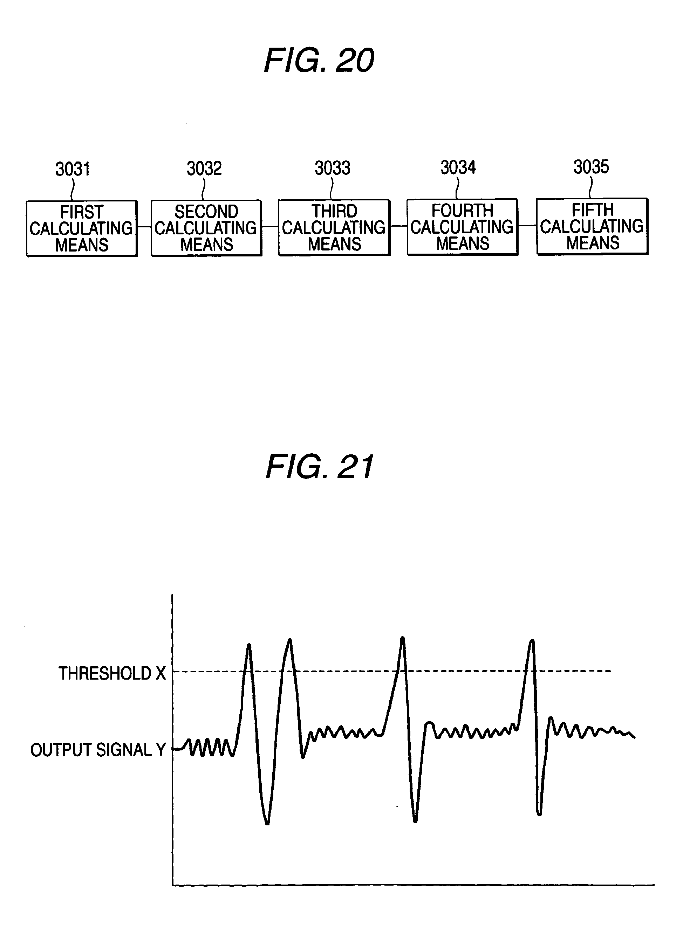

Fig. 20 is a block diagram for explaining a function related to the heartbeat information processing operation of the processing portion 5303. As shown in Fig. 20, the processing portion 5303 has first calculating means 3031, second calculating means 3032, third calculating means 3033, fourth calculating means 3034 and fifth calculating means 3035.

The first calculating means 3031 analog-to-digital converts each of a vibration signal output from the human body sensor 5011 and a vibration signal output from the car sensor 5023 into a digital value, and then calculates a moving average value successively within a certain time range and calculates the power spectrum of the time series data of the moving average value.

The second calculating means 3032 calculates a difference between both of the power spectra calculated by the first calculating means 3031 1 for each frequency. The third calculating means 3033 obtains, as an information about a heartbeat, a frequency at which the power spectrum is maximized in the preset fundamental frequency region of the heartbeat. The fourth calculating means 3034 multiplies the frequency obtained by the third calculating means 3033 by 60 and sets a value thus calculated to be a heart rate. Thus, the processing portion 5303 can obtain the heart rate (the information about a heartbeat) of a driver. In the embodiment, the fundamental frequency region of the heartbeat is set to be approximately 1 to 2 Hz. In this procedure, the heart rate is calculated every second to every ten seconds.

The fifth calculating means 3035 obtains a variation in the heart rate every second to every ten seconds by using the heart rate calculated by the fourth calculating means 3034. The fifth calculating means first calculates an average value and a standard deviation of the heart rate from time series data on the heart rate every second to every ten seconds, and divides the standard deviation of the heart rate by the average value of the heart rate to obtain the variation in the heart rate. For the information about the heartbeat, it is also possible to use both variations in the heartrate obtained by the fourth calculating means 3034 and the heart rate obtained by the fifth calculating means 3035.

The first to fifth calculating means are provided in a special IC (not shown). For the special IC, it is preferable to use a DSP (Digital Signal Processor), for example.

While the processing portion 5303 obtains the information about the heartbeat by taking note of the frequency band of approximately 1 to 2 Hz in the above description, moreover, it is possible to obtain an information about a breath by taking note of a frequency band of 1 Hz or less in the same manner.

Fig. 21 is a chart for explaining the body motion information processing operation of the processing portion 5303. In the processing portion 5303, there is obtained the number of times that a signal (an output signal Y) acquired by amplifying the output signal of the human body sensor 5011 at approximately 100 times by means of the first amplifier 5301 exceeds a predetermined threshold X per unit time. The output signal Y includes a vibration signal caused by the vibration of a vehicle itself in addition to a vibration signal caused by the biological activity (a heartbeat or a breath) of the driver. Since the body motion has a greater amplitude than the vibration of a car body, however, an information about a body motion can be obtained based on only the output signal of the human body sensor 5011.

The deciding portion 5305 decides the biological condition of the driver by referring to a biological information (an information about a heartbeat, an information about a breath and an information about a body motion) in a usual condition of the driver which are stored in the storage means 5040 based on the result of the processing of the processing portion 5303.

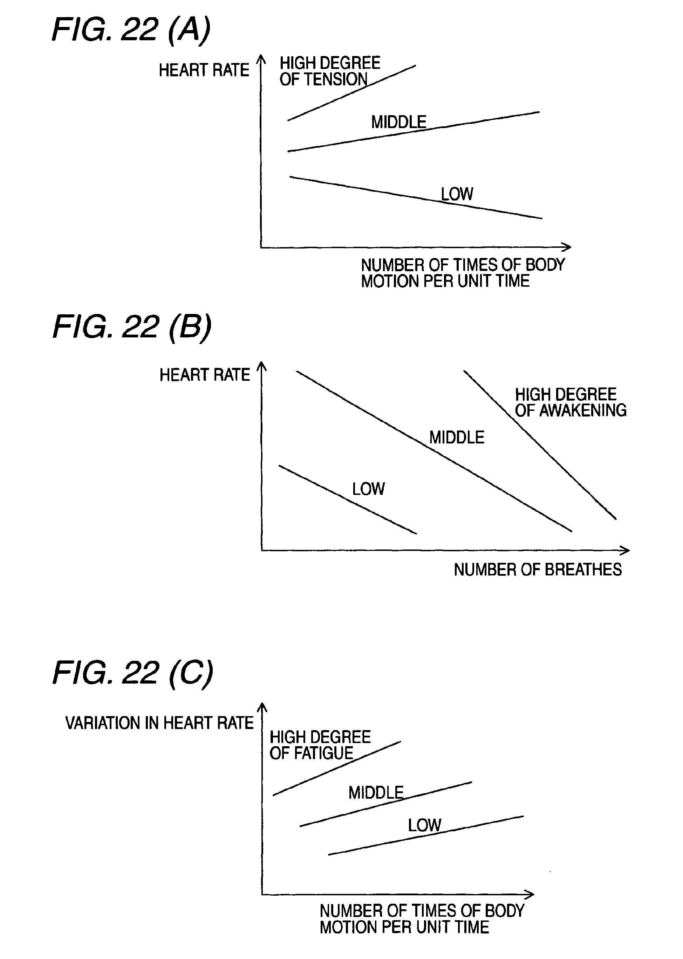

Fig. 22 is a chart for explaining a specific example of the decision of the biological condition which is made by the deciding portion 5305. Fig. 22(a) shows a correlation between the number of times of a body motion per unit time and a heart rate. The deciding portion 5305 decides a degree of tension based on the correlation. Fig. 22(b) shows a correlation between the number of breathes and the heart rate. The deciding portion 5305 decides a degree of awakening based on the correlation. Fig. 22(c) shows a correlation between the number of times of the body motion per unit time and a variation in the heart rate. The deciding portion 5305 decides a degree of fatigue based on the correlation. The correlations shown in Fig. 22 are based on the result of a measurement which is obtained by previously carrying out an experiment for the measured person, and it is assumed that these data are stored in the storage means 5040. For example, an accurate degree of awakening is measured from brain waves based on an experiment, and at the same time, data on the heartbeat, the breath and the body motion are collected to obtain a relationship between the degree of awakening and a heart rate, the number of breathes and a body motion pattern. At this time, it is desirable that a suitable relationship to all tastes should be obtained by permitting some freedom to a person to be inspected based on a large number of data.

In the decision of a degree of tension, as shown in Fig. 22(a), the degree of tension is decided in three stages of high, middle and low depending on any of tension degree curves obtained previously which is closer. The deciding portion 5305 compares the result of the processing of the processing portion 5303 (an information about a heartbeat and an information about a body motion) with that in this drawing and decides the degree of tension. For example, in the case in which a heart rate is high, it can be decided that the degree of tension is high if the number of times of the body motion is small and the degree of tension is middle if the number of times of the body motion is large.

In the decision of a degree of awakening, as shown in Fig. 22(b), the degree of awakening is decided in three stages of high, middle and low depending on any of awakening degree curves obtained previously which is closer. The deciding portion 5305 compares the result of the processing of the processing portion 5303 (the information about the heartbeat and the information about the breath) with that in this drawing and decides the degree of tension. For example, in the case in which the heart rate is high, it can be decided that the degree of awakening is middle if the number of breathes is small and the degree of awakening is high if the number of breathes is large.

In the decision of a degree of fatigue, as shown in Fig. 22(c), the degree of fatigue is decided in three stages of high, middle and low depending on any of fatigue degree curves obtained previously which is closer. The deciding portion 5305 compares the result of the processing of the processing portion 5303 (a variation in the heart rate and the number of times of the body motion) with that in this drawing and decides the degree of fatigue. In the case in which the variation in the heart rate is almost middle, for example, it can be decided that the degree of fatigue is high if the number of times of the body motion is small and the degree of fatigue is low if the number of times of the body motion is large.

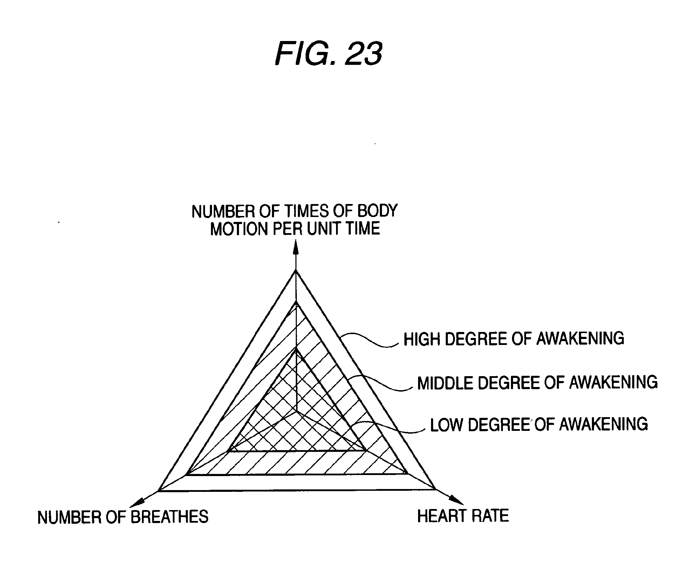

Although the deciding portion 5305 decides the degree of tension, the degree of awakening and the degree of fatigue in the three stages in the above description, it is also possible to make a decision in more stages such as five stages or ten stages if the correlation is previously obtained by an experiment. Moreover, it is also possible to decide the biological condition depending on a correlation between the variation in the heart rate and the number of breathes in addition to the correlation between the two elements (the heart rate and the number of times of the body motion). Furthermore, it is also possible to obtain a correlation among three elements of the heart rate, the number of breathes and the number of times of the body motion and to decide the degree of awakening as shown in an example of Fig. 23. Consequently, it is possible to make a decision more accurately. By adding an information about a body temperature to the correlation between the two elements or the three elements, furthermore, it is possible to make a decision with higher precision. It is possible to easily measure the body temperature by providing a body temperature sensor in a portion of a steering wheel with which a palm or a fingertip comes in contact, for example. Based on a body temperature rhythm (a circadian rhythm), moreover, it is also possible to correct the awakening degree curve.

While the description has been given to the processing of obtaining the information about a heartbeat, the information about a breath and the information about a body motion by the frequency analysis, it is also possible to decide the biological condition by using a signal waveform.

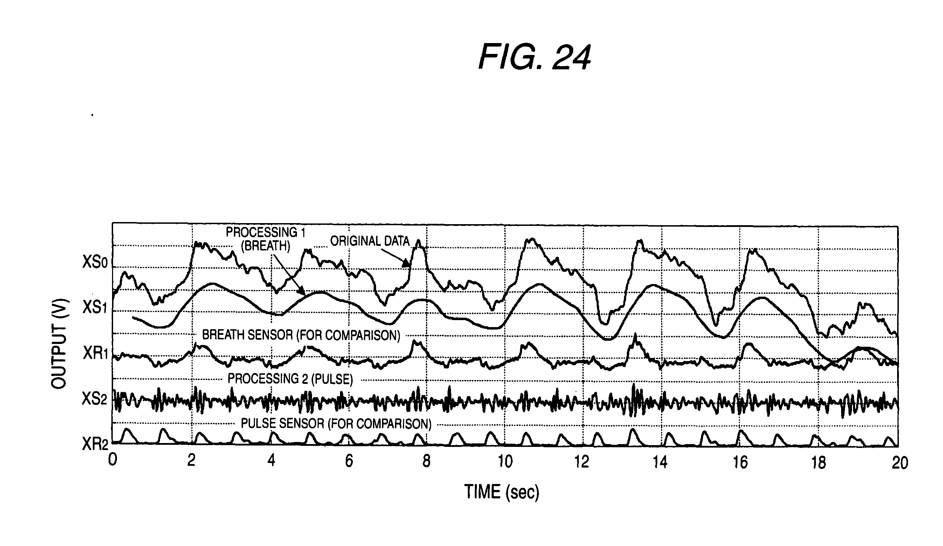

Fig. 24 is a chart for explaining a processing of deciding the biological condition by using a signal waveform. First of all, the phases of the output signal of the human body sensor 5011 and the output signal of the car sensor 5023 are coincident with each other to take a difference. As a result, a signal XS0 is obtained. More specifically, the signal XS0 represents a vibration signal which does not include a signal component caused by the vibration of a vehicle itself and is caused by the biological activity of a human body. Next, a frequency component of 0.1 to 1 Hz of the signal XS0 is extracted by a band-pass filter (Processing 1). As a result of the extraction, a signal XS1 is obtained and corresponds to the information about a breath. Moreover, a frequency component of 4 to 8 Hz (the resonant frequencies of the human body and the vehicle) of the signal XS0 is extracted by the band-pass filter (Processing 2). As a result of the extraction, a signal XS2 is obtained and corresponds to the information about a heartbeat. This processing can be carried out by the same processing portion as the processing portion 5303.

The signal values obtained as described above (the information about a heartbeat and the information about a breath) are compared with signal values for a comparison which are previously measured and stored in the storage means 5040 (for example, a signal for a breath is indicated as XR1 and a signal for a pulse is indicated as XR2). From the result of the comparison, the biological condition is decided in consideration of both the information about a heartbeat and the information about a breath. The biological condition can be thus decided by the same deciding portion as the deciding portion 5305.

In the decision of the biological condition, it is also possible to use another method of deciding the degree of tension based on a CVRR index or deciding the degree of fatigue based on a chaos index.

The driving control portion 5307 controls the driving operation of each portion of music supply means, scent supply means, oxygen supply means, air conditioning means, massage means and alarm means (not shown) which are provided in a vehicle based on the result of the decision obtained by the deciding portion 5305.

Description will be given to a specific example of the control of the driving control portion 5307. When it is decided that the degree of tension of a driver is increased by the deciding portion 5305, the driving control portion 5307 controls the driving operation of each portion in such a manner that the music supply means selects and gives such music as to slacken the tension, the scent supply means selects and supplies a scent (perfume) having a relaxing effect, the oxygen supply means supplies oxygen, and the massage means gives a massage to the waist or shoulder of a driver. The driving control portion 5307 regulates a volume, the intensity of the scent, the concentration of the oxygen, and the strength and speed of the massage stepwise depending on the degree of tension, for example.

When it is decided that the degree of fatigue of the driver is increased by the deciding portion 5305, moreover, the driving control portion 5307 controls the driving operation of each portion in such a manner that the music supply means selects and gives such music as to have the effect of healing the fatigue, the scent supply means selects and supplies a scent having the effect of healing the fatigue, the oxygen supply means supplies oxygen, and the massage means gives a massage to the waist or shoulder of a driver. The driving control portion 5307 regulates a volume, the intensity of the scent, the concentration of the oxygen, and the strength and speed of the massage stepwise depending on the degree of fatigue, for example.

When it is decided that the degree of awakening of the driver is reduced (the driver feels sleepy) by the deciding portion 5305, furthermore, the driving control portion 5307 controls the driving operation of each portion in such a manner that the music supply means selects and gives such music as to shake off sleepiness (music in a quick tempo), the scent supply means selects and supplies a scent having the effect of healing the fatigue, the oxygen supply means supplies oxygen, the massage means gives a massage to the waist or shoulder of a driver, the alarm means gives an alarm sound, and the air conditioning means supplies cold air. The driving control portion 5307 regulates a volume, the intensity of the scent, the concentration of the oxygen, the strength and speed of the massage, the volume of the alarm sound, and the set temperature of air conditioning stepwise depending on the degree of awakening, for example. In the case in which the degree of awakening of the driver is reduced, the alarm means may cause a display portion such as a speedometer in a car to be turned on and off or may give a notice to an external monitor center in addition to the generation of the alarm sound.

The control of the driving control portion 5307 will be collectively shown in Table 1.

### (Table 1)

Degree of tension
Degree of fatigue
Degree of awakening
Music
Scent
Oxygen
Vibration
Alarm
Air conditioning

As described above, in the seat device according to the embodiment, the biological information is detected by using the human body sensor 5011 and the car sensor 5023 which are formed by the piezoelectric cable sensor and the biological condition of the driver is decided based on the biological information. By this structure, it is possible to detect the biological information with a high sensitivity and high precision and to objectively decide the biological condition of the driver.

In the case in which a car is running, usually, a human body on a seat is excited (a frequency of 4 to 7 Hz) by a running vibration so that the biological information about the driver is detected with difficulty. In the embodiment, however, the processing portion 5303 carries out the frequency analysis over the output information of the two sensors (the human body sensor 5011 and the car sensor 5023) to calculate a difference between the power spectra, and obtains the information about a heartbeat by taking note of the fundamental frequency region of the heartbeat. Therefore, it is possible to detect the information about the heartbeat of a living body even if a vibration noise is made on an outside. By this structure, moreover, it is possible to easily obtain the information about the heartbeat without taking the phase shift of the output informations of the two sensors into consideration.

The control means or the storage means may be provided in a vehicle body or may be provided in a control center disposed apart from the vehicle. In the case in which the control means and the vehicle are provided apart from each other, the output informations of the human body sensor 5011 and the car sensor 5023 are transmitted by wireless.

While the biological condition of the driver is decided in the embodiment, moreover, the decision can be implemented for a person getting on a passenger seat or a rear seat other than the driver in the same manner.

### (Eighth Embodiment)

In the case in which a child is to get on a vehicle, it is obliged to be put on a child seat. In particular, it can be supposed that a baby that is less than one year old is desirably caused to get on a special child seat for the baby to be put on a rear seat in respect of a safety. When the child seat is put on the rear seat, however, the situation of the baby is grasped from a driver's seat or a passenger seat with difficulty. In the embodiment, therefore, description will be given to a child seat capable of informing a driver of the biological condition of the baby and causing the baby to spend time comfortably in the case in which the baby is caused to get on the child seat to be put on the rear seat.

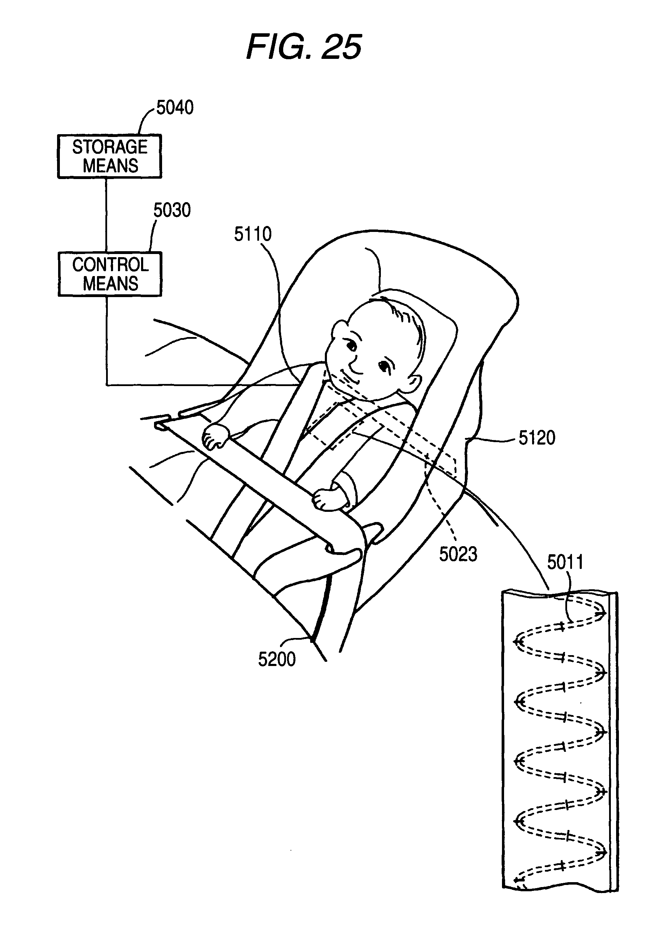

Fig. 25 is a view showing the structure of a child seat, illustrating an eighth embodiment of the biological condition deciding device according to the invention. In the embodiment, description will be given to the case in which the biological information of a baby (an information about a heartbeat, an information about a breath and an information about a body motion) getting on the child seat is detected to decide a biological condition. In Fig. 25, the same portions as those in Fig. 8 described in the seventh embodiment have the same reference numerals.

A child seat shown in Fig. 25 comprises a child seat body 5120 having a car sensor 5023 buried on an outer surface, and a child seat belt 5110 formed integrally with a human body sensor 5011, and is connected to control means 5030 and storage means 5040. The child seat is mounted toward the rear side of a vehicle and is fixed by stretching a seat belt 5200 for a rear seat which is provided on the rear seat over the child seat body 5120. The child seat belt 5110 is stretched from the back side of both shoulders of the baby to a portion between both legs through the front face of a body, thereby fixing the body of the baby to the child seat body 5120.

While the body sensor 5011 is formed integrally with the child seat belt 5110 in the embodiment, the human body sensor 5011 may be buried in a position on the inside of the child seat body 5120 in which it comes in contact with the head portion of the baby or a head pad burying the human body sensor therein may be caused to internally touch the child seat body 5120. If the head pad is provided, the head portion of the baby can be fixed more reliably, and furthermore, the human body sensor adheres to the head portion of the baby. Therefore, it is possible to sense the biological informations of the baby (the information about a heartbeat, the information about a breath and the information about a body motion) more accurately.

The embodiment is different from the seventh embodiment in respect of the contents of the control of a driving control portion 5307. This respect will be described below.

The driving control portion 5307 controls the driving operation of each portion of AV (Audio Visual) supply means, scent supply means, oxygen supply means, air conditioning means, vibrating means and informing means (which are not shown) provided in the child seat based on the result of the decision obtained by a deciding portion 5305.

When it is decided by the deciding portion 5305 that a baby is set in a tension condition or a body motion becomes violent and the baby is fretful, the driving control portion 5307 controls the driving operation of each portion in such a manner that the vibrating means vibrates the child seat body 5120 to give a comfortable vibration to the baby, the scent supply means selects and supplies a scent having a relaxing effect, the oxygen supply means supplies oxygen, the AV supply means supplies music having the relaxing effect or a similar sound to a human cardiac sound and displays a dynamic image such as the video of a mother or an animation on the backrest of a rear seat, and the air conditioning means supplies air at a suitable temperature. The driving control portion 5307 regulates a volume, the intensity of a scent, the concentration of oxygen, a pattern for vibrating the child seat body 5120 (how to swing) or a speed stepwise depending on the degree of tension of the baby, for example

In general, the baby is fretful due to a rise in a temperature in the child seat in many cases. Therefore, it is preferable that the air conditioning means should supply air at a lower temperature than a room temperature. The scent supply means, the oxygen supply means and the air conditioning means supply the scent, the oxygen or the air from a supply port provided on the outer edge of the child seat body 5120 in order not to hit the body of the baby.

Moreover, the music, the video and the perfume that the baby likes may be preset or an air temperature or a humidity may be predetermined for the supply. By displaying the video of the face of a mother, it is possible to obtain an advantage of relaXing the baby and causing it to feel easy. In the case in which the baby is too young to recognize the face of the mother, moreover, it is possible to obtain the same advantages as those in the display of a human face even if two black points are displayed at an interval of approximately 10 cm.

In the case in which the driving control portion 5307 detects, by the deciding portion 5305, a change in a biological condition, for example, a state in which the baby has a desire to urinate, the informing means controls the driving operation to generate predetermined sounds or music. Consequently, other passengers can be informed of the biological condition of the baby.

The AV supply means, the scent supply means, the oxygen supply means, the air conditioning means and the informing means may be provided in a vehicle in place of the child seat.

As described above, according to the child seat in accordance with the embodiment, the biological condition of a baby is decided to drive each support means. Therefore, the baby can spend time comfortably in a vehicle. According to the child seat in accordance with the embodiment, moreover, the baby is rarely fretful with a feeling of discomfort due to a rise in a temperature. Even if the child seat is put on the rear seat, therefore, a driver can easily concentrate on driving without absorbing himself (herself) in the situation of the baby. As a result, the driver can carry out the driving more safely.

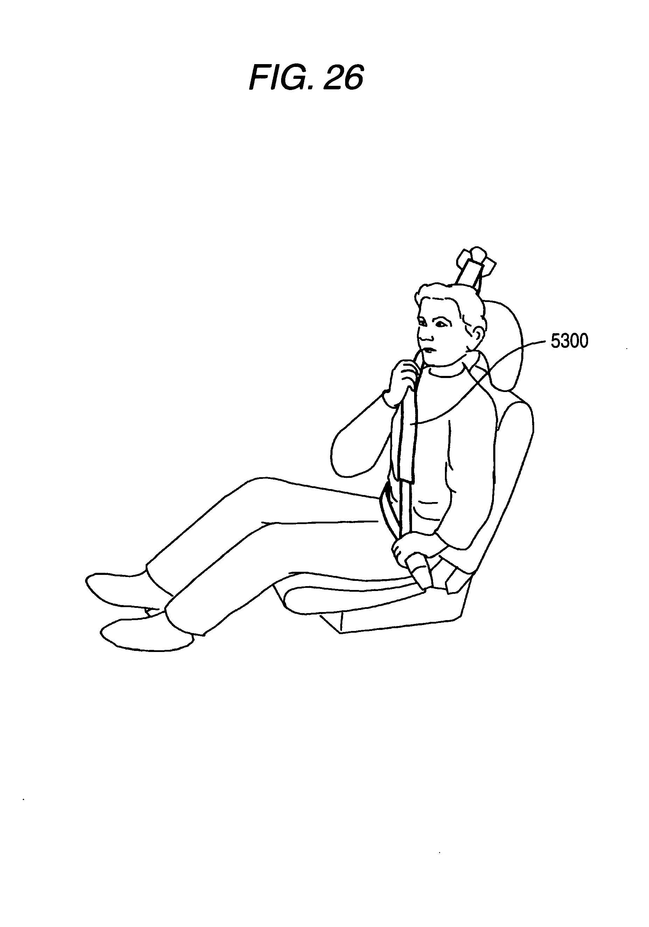

While the description has been given to the case in which the seat belt and the piezoelectric cable sensor (the human body sensor 5011) are integrated with each other, it is also possible to employ a structure in which the piezoelectric cable sensor is inserted and attached to a stopper and is thus drawn and extended together with a buckle engaging the seat belt to come in contact with the chest portion of the driver when the buckle is pulled. As shown in Fig. 26, moreover, it is possible to detect the micro signal of a human body even if the piezoelectric cable sensor is provided in a seat belt pad 5300 for relieving a feeling of discomfort caused by the direct contact of the seat belt with a shoulder, a nape or a waist portion, and the same advantages can be thus obtained.

While the human body sensor and the car sensor are constituted by the piezoelectric cable sensor in the seventh and eighth embodiments, the invention can be implemented in the same manner even if they are constituted by a sheet-shaped or band-shaped piezoelectric sensor.

### (Ninth Embodiment)

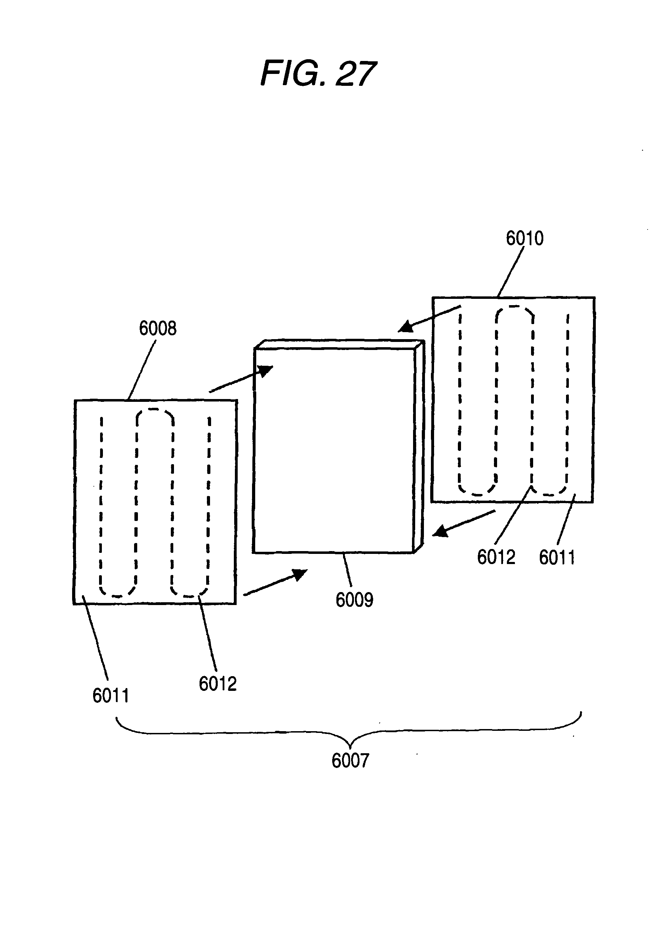

Fig. 27 shows a view showing the structure of a sensing unit 6007 in a biological information detecting device according to a ninth embodiment of the invention. In the embodiment, description will be given to an example in which the heartbeat of a human body on the seat of a car is detected by using the biological information detecting device according to the invention.

In Fig. 27, the sensing unit 6007 comprises first vibration detecting means 6008, vibration attenuating means 6009 and second vibration detecting means 6010. The first vibration detecting means 6008 serves to detect the vibration of a living body and a cable-shaped piezoelectric sensor 6012 having a flexibility is provided in a meandering form on a foundation cloth 6011 formed by a nonwoven fabric or a sponge sheet.

The vibration attenuating means 6009 is formed by an elastic member having such a characteristic as to attenuate at least a vibration in the fundamental frequency region of a heartbeat. For such an elastic member, it is preferable to use a sponge sheet having a greater thickness than the foundation cloth 6011, for example.

The second vibration detecting means 6010 has the same structure as that of the first vibration detecting means 6008. The first vibration detecting means 6008 and the vibration attenuating means 6009 are bonded and integrated with each other, and the second vibration detecting means 6010 and the vibration attenuating means 6009 are bonded and integrated with each other.

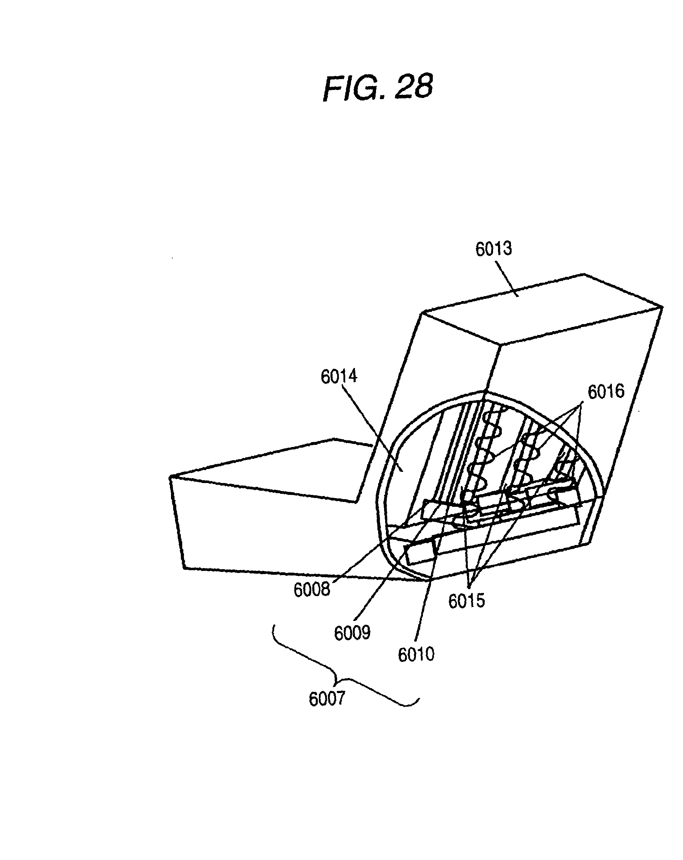

Fig. 28 is a view showing the case in which the sensing unit 6007 is provided on a seat 6013 in a car, a main part being taken away. In the embodiment, the sensing unit 6007 is provided in the back side of the seat 6013. The first vibration detecting means 6008 is disposed in contact with the back face side of the seat of a cushion portion 6014 provided in the back face of the seat, and the second vibration detecting means 6010 includes, on a surface thereof, a spacer 6015 formed of an elastic member and is always provided in such a position as to receive pressing by a seat spring 6016 of the seat 6014 and a cooperating member thereof. More specifically, the sensing unit 6007 is interposed between the cushion portion 6014 provided in the back face of the seat and the seat spring 6016 through the spacer 6015.

It is preferable that the sensing unit 6007 should have such a shape as to cover the whole cushion portion 6014 on the back face of the seat. However, it is desirable that the sensing unit 6007 should be provided within a range from a position corresponding to the waist part of a human body to a position corresponding to at least the lowermost part of a shoulder blade during seating in the cushion portion 6014 on the back face of the seat in consideration of a portion in which the human body adheres to the back face of the seat better in a seating condition.

Moreover, the sensing unit 6007 is provided on the back face of the seat in such a manner that a straight part in the meandering portion of the piezoelectric sensor 6012 is almost parallel with the vertical direction of the seat 6013.

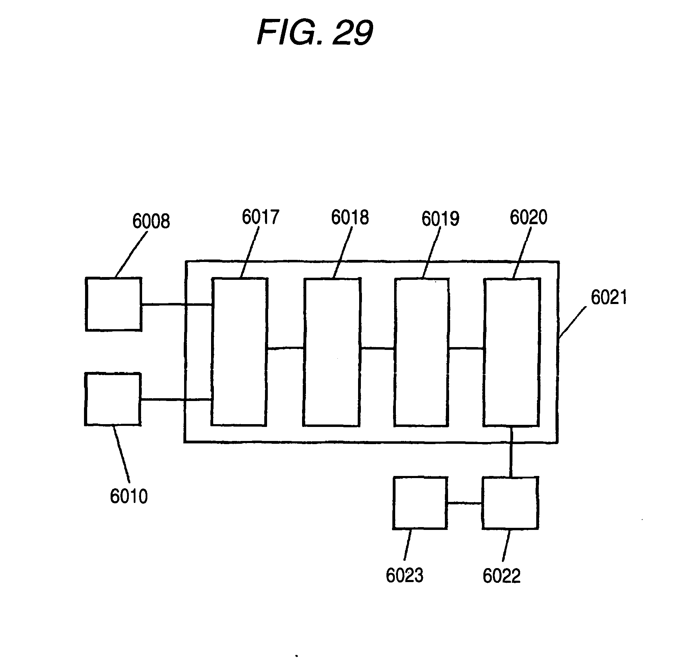

Fig. 29 is a block diagram showing the biological information detecting device according to the embodiment. First calculating means 6017 analog-to-digital converts each of the output signals of the first vibration detecting means 6008 and the second vibration detecting means 6010 into a digital value, and then calculates a moving average value successively within a certain time range and calculates the power spectrum of the time series data of the moving average value.

Second calculating means 6018 calculates a difference between both of the power spectra calculated by the first calculating means 6017 for each frequency. Third calculating means 6019 obtains, as an information about a heartbeat, a frequency at which the power spectrum is maximized in the fundamental frequency region of the heartbeat which is preset based on the result of the calculation of the second calculating means 6018. Fourth calculating means 6020 multiplies the frequency obtained by the third calculating means 6019 by 60 and sets a value thus calculated to be a heart rate.

The first to fourth calculating means are provided in a special IC 6021. For the special IC 6021, it is preferable to use a DSP (Digital Signal Processor), for example.

If necessary, moreover, it is also possible to carry out a control in order to display a heart rate obtained by the fourth calculating means 6019 on a display portion 6023 by control means 6022. The display portion 6023 may have such a structure that a special display portion for a front panel in a car is provided or a display is carried out on the display of a navigation device, for example.

Explanation will be given to the operation and function of the biological information detecting device having the structure described above.

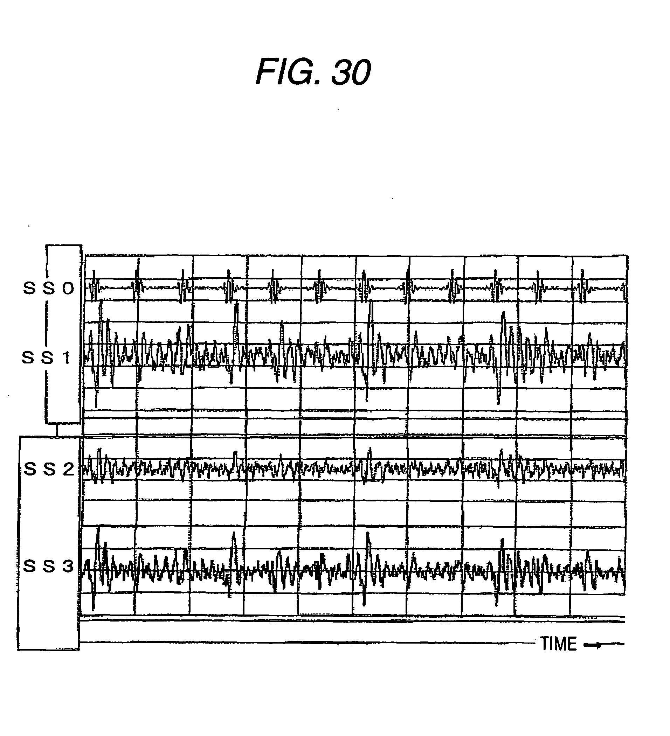

First of all, referring to the case in which a car is set in an idling state, Fig. 30 is a characteristic chart representing the temporal transition of an electrocardiogram SS0 of a human body on a seat, an output signal SS1 of the first vibration detecting means 6008, an output signal SS2 of the second vibration detecting means 6010, and a difference SS3 between SS1 and SS2.

As shown in Fig. 30, a synchronous signal with a heartbeat which is clearly recognized at SS0 can be seen in some places of SS1 and SS2 even if the sensing unit 6007 is provided on the back side of the seat of the cushion portion 6014 in place of a surface on the human body side of the back face of the seat. Accordingly, it is possible to detect a heart rate by a well-known method of calculating the autocorrelation function of data of SS1 to obtain a heart rate in the idling state depending on the vibrating characteristic or aging of the car for example.

The main vibrating component of the output was approximately 4 to 7 Hz to be the resonant frequency of a living body.

Moreover, SS3 was obtained as disclosed in the Patent Document 3. Also in this case, a synchronous signal with a heartbeat was recognized in some places, which were slightly obscure.

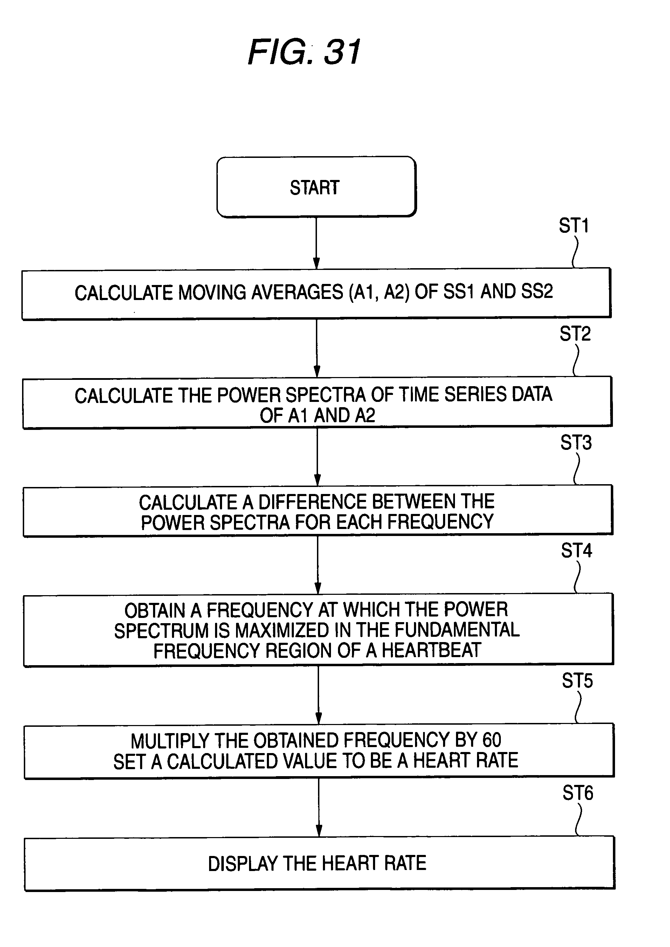

Next, a method of detecting a heart rate by the above structure will be described with reference to Fig. 31. Fig. 31 1 is a flowchart showing the detection of a heart rate in the biological information detecting device according to the embodiment.

First of all, at Step ST1, the first calculating means 17 analog-to-digital converts each of the output signals SS1 and SS2 of the first vibration detecting means 6008 and the second vibration detecting means 6010 into a digital value, and then calculates moving average values A1 and A2 successively within a certain time range.

At Step ST2, next, the first calculating means 6017 calculates the power spectrum of the time series data of A1 and A2.

At Step ST3, then, the second calculating means 6018 calculates a difference between the power spectra of both SS1 and SS2 calculated by the first calculating means 6017 for each frequency.

At Step ST4, thereafter, the third calculating means 6019 obtains, as an information about a heartbeat, a frequency at which the power spectrum is maximized in the fundamental frequency region of the heartbeat which is preset based on the result of the calculation of the second calculating means 6018.

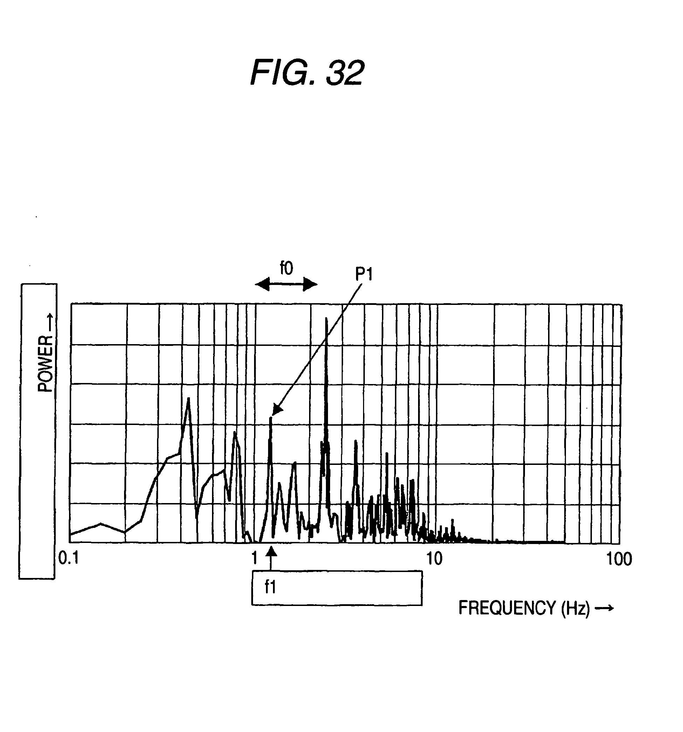

A procedure at this time will be described with reference to Fig. 32. Fig. 32 is a characteristic chart showing the difference between the power spectra of both SS1 and SS2 calculated by the second calculating means 6018 at the Step ST3. In Fig. 32, a clear peak can be recognized in a frequency region f0 of approximately 1 to 2 Hz to be the fundamental frequency region of a heartbeat and a frequency at the peak is coincident with the frequency of a heartbeat calculated from SS0. Accordingly, a frequency f1 at which the difference between the power spectra is maximized to be P1 in the frequency region f0 of approximately 1 to 2 Hz to be the fundamental frequency region of the heartbeat can be obtained as an information about a heartbeat, that is, the frequency of the heartbeat based on the characteristic shown in Fig. 32.

When f1 is obtained, the fourth calculating means 6020 multiplies, by 60, the frequency obtained by the third calculating means 6019 and sets a value thus calculated to be a heart rate at Step ST5. Then, the heart rate obtained at Step ST6 is displayed on the display portion 6023.

The processing at the Steps ST1 to ST6 is repeated every predetermined unit time and the heart rate is detected every unit time.

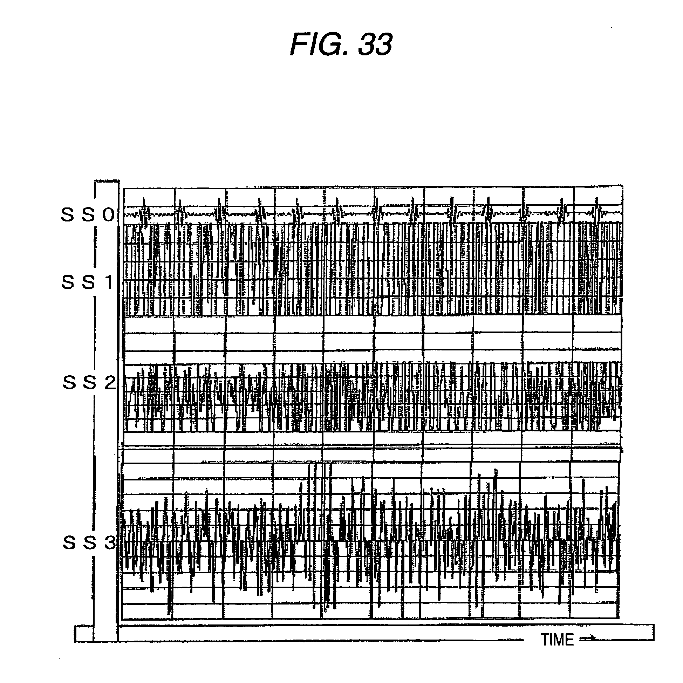

Referring to the case in which the car is set in a running state, next, Fig. 33 is a relational chart representing the temporal transition of an electrocardiogram SS0 of a human body on a seat, an output signal SS1 of the first vibration detecting means 6008, an output signal SS2 of the second vibration detecting means 6010, and a difference SS3 between SS1 and SS2.

As shown in Fig. 33, the human body on the seat is excited by a running vibration and such great outputs as to saturate both of the signals SS1 and SS2 are generated from the first vibration detecting means 6008 and the second vibration detecting means 6009. The main vibration component of the output was approximately 4 to 7 Hz to be the resonant frequency of a living body.

Accordingly, a synchronous signal with a heartbeat in the idling state shown Fig. 30 is buried in a signal component generated by the excitation of a body through a running vibration so that SS1 and SS2 cannot be confirmed.

Also at SS3 to be the difference between SS1 and SS2, moreover, the synchronous signal with the heartbeat in the idling state is recognized in some places and is not recognized at all in Fig. 33.

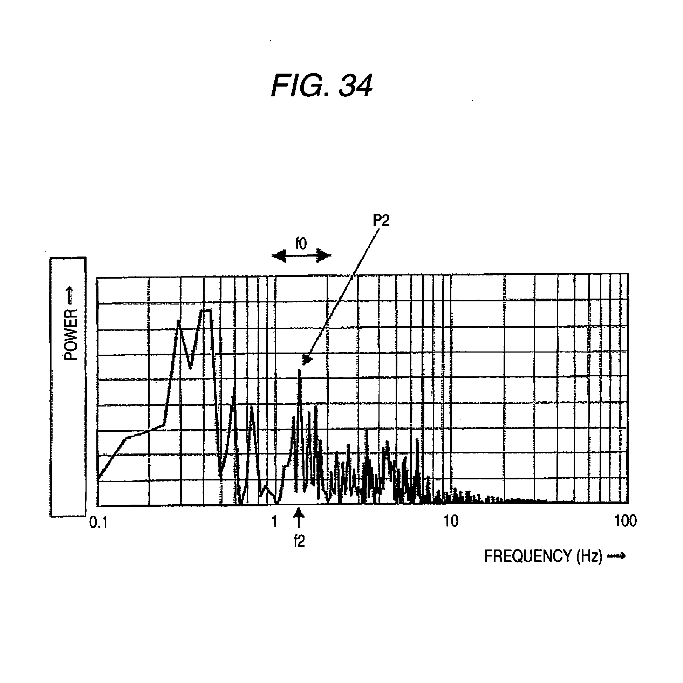

When the processing at the Steps ST1 to ST3 is carried out based on the processing procedure shown in Fig. 31, therefore, the difference between the power spectra of both SS1 and SS2 is obtained as shown in Fig. 34. More specifically, from Fig. 34, a clear peak was recognized also in this case in the frequency region f0 of approximately 1 to 2 Hz to be the fundamental frequency region of a heartbeat, and a frequency at this peak was coincident with the frequency of a heartbeat calculated from SS0. Based on the characteristic shown in Fig. 34, accordingly, it is possible to obtain a frequency f2 at which the difference between the power spectra is maximized to be P2 in the frequency region f0 of approximately 1 to 2 Hz to be the fundamental frequency region of a heartbeat as the information about the heartbeat, that is, the frequency of the heartbeat.

When f2 is obtained, the fourth calculating means 6020 multiplies, by 60, the frequency obtained by the third calculating means 6019 and sets a value thus calculated to be a heart rate at the Step ST5. Then, the heart rate obtained at Step ST6 is displayed on the display portion 6023.

The processing at the Steps ST1 to ST6 is repeated every predetermined unit time and the heart rate is detected every unit time.

As described above, in the embodiment, the difference between the power spectra of the two vibration detecting means is calculated and the information about a heartbeat is obtained by taking note of the fundamental frequency region of the heartbeat in place of the frequency band of the resonant frequency of the living body. Therefore, it is possible to provide a biological information detecting device having a high utility which can detect an information about the heartbeat of the living body even if a vibration noise is made on an outside.

Moreover, the sensing unit 6007 is molded like the sheet and can detect a vibration within a wide range based on a heartbeat propagated from the living body on the seat to the back face side of the seat. Consequently, it is possible to enhance precision in the detection of the information about the heartbeat. Moreover, the first vibration detecting means 6008 is disposed in contact with the back face side of the seat of the cushion portion 6014 provided in the back face of the seat. Therefore, a feeling of incompatibility is not given to seating on the back face side of the seat. Furthermore, the second vibration detecting means 6010 is provided with the spacer 6015 formed by an elastic member on a surface, and the running vibration is attenuated by the spacer when it is propagated to the second vibration detecting means 6010 so that an unnecessary vibration is propagated to the second vibration detecting means 6010 or the first vibration detecting means 6008 with difficulty. Consequently, the precision in the detection of the information about the heartbeat can further be enhanced.

In addition, the cable-shaped piezoelectric sensor 6012 having a flexibility is used. Therefore, the degree of freedom of a provision can be enhanced, for example, a bending provision can be carried out, and furthermore, the straight part in the meandering portion of the piezoelectric sensor 6012 is provided to be almost parallel with the vertical direction of the seat 6007. Even if the vibration in the vertical direction to be the main vibrating component of the running vibration is received, therefore, the piezoelectric sensor 6012 is deformed with difficulty and is influenced by the running vibration with difficulty. Consequently, it is possible to enhance precision in detection.

Furthermore, the vibration attenuating means 6009 has such a characteristic as to attenuate at least a vibration in the fundamental frequency region of the heartbeat. When the heartbeat vibration is propagated from the first vibration detecting means 6008 to the second vibration detecting means 6010, the vibration attenuating means 6009 attenuates at least the vibration in the fundamental frequency region of the heartbeat. Therefore, a difference in a power spectrum between the respective output signals of the first vibration detecting means 6008 and the second vibration detecting means 6010 in the fundamental frequency region of the heartbeat can be caused to be clearer. Therefore, it is possible to further enhance the precision in the detection of the information about the heartbeat.

Moreover, the first calculating means 6017 serves to successively calculate a moving average value within a certain time range for each of the output signals of the first vibration detecting means 6008 and the second vibration detecting means 6010 and to calculate the power spectrum of the time series data of the moving average value. By calculating the moving average value, it is possible to remove an unnecessary vibration component which is caused by the running vibration. Therefore, it is possible to further enhance the precision in the detection of the information about the heartbeat.

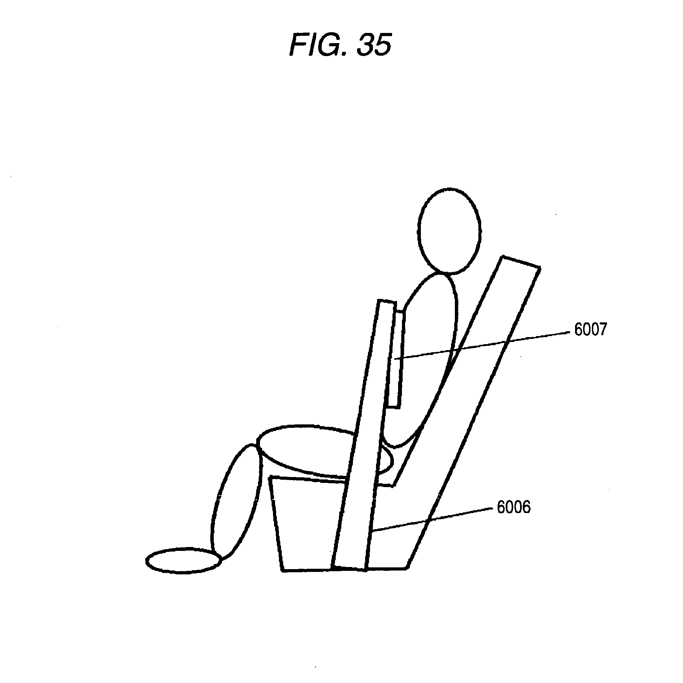

While the sensing unit 6007 is provided on the back face of a seat in a car in the ninth embodiment, the sensing unit 6007 can have a structure in which the first vibration detecting means 6008, the vibration attenuating means 6009 and the second vibration detecting means 6010 are molded integrally like a sheet, and is provided between a seat belt 6006 of the car and a living body on the seat as shown in Fig. 35. Since they are molded like the sheet, it is possible to detect a vibration based on a heartbeat within a wide range between the seat belt and the living body on the seat. Consequently, it is possible to enhance precision in the detection of the information about the heartbeat.

Moreover, it is also possible to employ a structure in which an object to come in contact with the first vibration detecting means 6008 is at least one of objects with which a part of a human body to be a living body comes in contact for living, for example, clothes, a bracelet, a belt, a necklace, a chair, a toilet seat, a bathtub, scales, bedclothes, a seat for a vehicle, a floor, a cell phone and a PDA, and the first vibration detecting means is provided in various objects to be used in a daily life. Therefore, it is possible to obtain the information about a heartbeat everywhere at any time so that a convenience can be enhanced.

Furthermore, the first vibration detecting means and the second vibration detecting means are not restricted to the piezoelectric sensor but may be another vibration sensor such as an electrostatic capacity type vibration sensor or strain gauge, or an optical fiber type vibration sensor.

While the invention has been described in detail with reference to the specific embodiments, it is apparent to the skilled in the art that various changes and modifications can be made without departing from the spirit and scope of the invention.

The application is based on Japanese Patent Application No. 2003-309797 filed on September 2, 2003, Japanese Patent Application No. 2003-331639 filed on September 24, 2003, and Japanese Patent Application No. 2003-333857 filed on September 25, 2003, and contents thereof are incorporated herein by reference.

### <Industrial Applicability>

A biological sensor according to the invention is attached to or is held as a single unit in at least one of objects with which a part of a human body to be a living body can come in contact for living, for example, clothes, a bracelet, a belt, a necklace, a chair, a toilet seat, a bathtub, scales, bedclothes, a seat for a vehicle, a floor, a cell phone and a PDA, and consequently, can easily be provided on various objects to be used in a daily life. Thus, a biological information such as an information about a heartbeat, an information about a body motion or an information about a breath can be obtained everywhere at any time, and this countermeasure can be implemented so that an effective life support can be realized.

## Claims

1. A biological sensor comprising:
acceleration detecting means constituted to detect an acceleration including a vibration of a body surface of a person to be measured; and
deciding means for extracting at least two of an information about a heartbeat, an information about a body motion and an information about a breath from an information output from the acceleration detecting means and deciding a biological condition of the measured person.

2. The biological sensor according to claim 1, further comprising:
storage means for storing the information output from the acceleration detecting means,
the deciding means setting the information output from the acceleration detecting means which is stored in the storage means to be a reference information and deciding the biological condition in consideration of the reference information.

3. The biological sensor according to claim 1 or 2, wherein the storage means serves to store the information output from the acceleration detecting means in a usual condition of the measured person, and
the deciding means serves to decide the biological condition of the measured person by referring to the output information of the storage means.

4. The biological sensor according to any of claims 1 to 3, wherein the acceleration detecting means includes an elastic member having a low repulsion and a piezoelectric cable sensor laid on the elastic member.

5. The biological sensor according to any of claims 1 to 4, wherein the acceleration detecting means is a piezoelectric cable sensor attached to a keyboard of a personal computer and laid on a palm rest provided in such a manner that at least a part of a palm of the measured person comes in contact therewith.

6. The biological sensor according to any of claims 1 to 4, wherein the acceleration detecting means is a piezoelectric cable sensor laid on a seat portion.

7. The biological sensor according to any of claims 1 to 4, wherein the acceleration detecting means is a piezoelectric cable sensor laid on a pendant to be hung around a neck of the measured person.

8. The biological sensor according to any of claims 1 to 4, wherein the acceleration detecting means is a piezoelectric cable sensor laid on a ring-shaped member formed to be attachable to the measured person.

9. The biological sensor according to any of claims 1 to 4, wherein the acceleration detecting means is a piezoelectric cable sensor laid on a bed.

10. The biological sensor according to any of claims 1 to 4, wherein the acceleration detecting means is a piezoelectric cable sensor laid on a toilet seat in a bathroom.

11. An autonomic nerve training system using the biological sensor according to any of claims 1 to 10, wherein the deciding means serves to detect an autonomic nerve condition from at least two of the heartbeat, the body motion and the breath,
the autonomic nerve training system further comprising:
display means for displaying the autonomic nerve condition as an information output from the deciding means in such a manner that the trainee carries out an autonomic nerve training while seeing a display of the display means.

12. An aromatherapy system using the biological sensor according to any of claims 1 to 10, wherein the deciding means serves to detect a degree of tension as an output information, and
a perfume is selected by referring to an aromatherapy information depending on the degree of tension, thereby relieving a biological condition.

13. A sound system using the biological sensor according to any of claims 1 to 10, wherein the deciding means serves to detect a degree of tension,
the sound system comprising music supply means for selecting and supplying proper music depending on the degree of tension.

14. A nap preventing system using the biological sensor according to any of claims 1 to 10, wherein the deciding means serves to detect a degree of awakening,
the nap preventing system comprising:
support means for supporting awakening; and
driving control means for controlling a driving operation of the support means depending on the degree of awakening.

15. A nursing support system using the biological sensor according to any of claims 1 to 10, wherein the deciding means serves to detect a desire to urinate,
the nursing support system comprising:
display means for displaying the output information for a nursing person.

16. An input support system using the biological sensor according to any of claims 1 to 10, wherein the deciding means serves to detect a degree of awakening of a brain, and
an activation of a screen saver is controlled based on the degree of awakening.

17. A biological condition deciding device for deciding a biological condition of a person to be measured based on an information output from a piezoelectric sensor having a flexibility, comprising:
a first piezoelectric sensor provided on a seat belt;
a second piezoelectric sensor provided in a position placed apart from the measured person; and
deciding means for deciding the biological condition of the measured person based on an information output from each of the first and second piezoelectric sensors,
the first piezoelectric sensor serving to detect at least two of an information about a heartbeat, an information about a body motion and an information about a breath of the measured person and the second piezoelectric sensor serving to detect an information about a vibration of a vehicle.

18. The biological condition deciding device according to claim 17, wherein the first piezoelectric sensor is provided in a position on the seat belt which comes in contact with the measured person.

19. The biological condition deciding device according to claim 17 or 18, wherein the first piezoelectric sensor is formed integrally with the seat belt.

20. The biological condition deciding device according to any of claims 17 to 19, wherein the deciding means has a processing portion for processing the information output from each of the first and second piezoelectric sensors,
the processing portion serving to carry out a frequency analysis over the output information.

21. The biological condition deciding device according to claim 20, wherein the processing portion has first calculating means for calculating power spectra of respective output signals of the first and second piezoelectric sensors, second calculating means for calculating, for each frequency, a difference between both of the power spectra calculated in the first calculating means, and third calculating means for obtaining, as an information about a heartbeat, a frequency at which the difference between the power spectra is maximized in a fundamental frequency region of the heartbeat which is preset based on a result of the calculation of the second calculating means.

22. The biological condition deciding device according to any of claims 17 to 21, further comprising storage means for storing the information output from each of the first and second piezoelectric sensors,
the deciding means serving to decide the biological condition of the measured person by referring to an information stored in the storage means.

23. The biological condition deciding device according to claim 22, wherein the storage means serves to store the information output from the sensor in a usual condition of the measured person.

24. The biological condition deciding device according to any of claims 17 to 23, wherein the first piezoelectric sensor is provided in a meandering form.

25. A sound system using the biological condition deciding device according to any of claims 17 to 24, wherein the deciding means serves to decide a degree of tension of the measured person,
the sound system comprising music supply means for selecting and supplying proper music depending on the degree of tension.

26. A fatigue relaxing support system using the biological condition deciding device according to any of claims 17 to 24, wherein the deciding means serves to decide a degree of fatigue of the measured person,
the fatigue relaxing support system comprising:
support means for supporting a relaxation of fatigue; and
driving control means for controlling a driving operation of the support means depending on the degree of fatigue.

27. A nap preventing system using the biological condition deciding device according to any of claims 17 to 24, wherein the deciding means serves to decide a degree of awakening of the measured person,
the nap preventing system comprising:
support means for supporting awakening; and
driving control means for controlling a driving operation of the support means depending on the degree of awakening.

28. A biological information detecting device comprising first vibration detecting means provided directly on a living body or provided on an object coming in contact with the living body and serving to detect a vibration of the living body, second vibration detecting means opposed to the first vibration detecting means and provided through vibration attenuating means having a preset vibration attenuating characteristic, first calculating means for calculating power spectra of respective output signals of the first vibration detecting means and the second vibration detecting means, second calculating means for calculating, every frequency, a difference between both of the power spectra calculated by the first calculating means, and third calculating means for obtaining, as an information about a heartbeat, a frequency at which the difference between the power spectra is maximized in a fundamental frequency region of the heartbeat which is preset based on a result of the calculation of the second calculating means.

29. The biological information detecting device according to claim 28, wherein the contact object is at least one of objects with which a part of a human body to be a living body can come in contact for living, for example, clothes, a bracelet, a belt, a necklace, a chair, a toilet seat, a bathtub, scales, bedclothes, a seat for a vehicle, a floor, a cell phone and a PDA.

30. The biological information detecting device according to claim 28, wherein the first vibration detecting means, the vibration attenuating means and the second vibration detecting means are molded integrally like a sheet and are provided between a seat belt of a car and a living body on a seat.

31. The biological information detecting device according to claim 28, wherein the first vibration detecting means, the vibration attenuating means and the second vibration detecting means are molded integrally like a sheet and are provided in a back side of a seat, the first vibration detecting means is disposed in contact with a back side of the seat of a cushion portion provided in a back face of the seat, and the second vibration detecting means includes a spacer formed by an elastic member on a surface.

32. The biological information detecting device according to claim 30 or 31, wherein the first vibration detecting means and the second vibration detecting means are formed with a cable-shaped piezoelectric sensor having a flexibility provided on a foundation cloth in a meandering form, and a straight part in a meandering portion of the piezoelectric sensor is provided in almost parallel with a vertical direction of the seat.

33. The biological information detecting device according to any of claims 28 to 32, wherein the vibration attenuating means has such a characteristic as to attenuate at least a vibration in a fundamental frequency region of a heartbeat.

34. The biological information detecting device according to any of claims 28 to 33, wherein the first calculating means successively calculates a moving average value within a certain time range for each of the output signals of the first vibration detecting means and the second vibration detecting means and calculates a power spectrum of time series data of the moving average value.

35. A biological information detecting device, wherein a plurality of vibration detecting means provided directly on a living body or provided on an object coming in contact with the living body and serving to detect a vibration of the living body is disposed through vibration attenuating means having a certain vibration attenuating characteristic, and frequency components of respective outputs are obtained and a frequency at which a difference is maximized in a fundamental frequency region of a heartbeat is acquired as an information about the heartbeat.

36. A biological information detecting device comprising first vibration detecting means for detecting a vibration of a living body and second vibration detecting means opposed to the first vibration detecting means and disposed through vibration attenuating means having a vibration attenuating characteristic, a frequency component of an output signal of each of the first vibration detecting means and the second vibration detecting means being obtained and a frequency at which a difference is maximized in a fundamental frequency region of a heartbeat being acquired as an information about the heartbeat.
